# EUROPEAN PATENT APPLICATION

(11) **EP 3 929 311 A2**
(43) Date of publication of application: **29.12.2021**
(21) Application number: 21159409.8
(22) Date of filing: 25.02.2021
(51) Int. Cl.: C12Q 1/70

(54) **METHODS AND MEANS FOR CORONA VIRUS NUCLEIC ACID DETECTION**

(30) Priority: 26.06.2020 EP 20182595
(62) Divisional of application: 21198236.8
(71) Applicant: Fraunhofer Gesellschaft zur Förderung der angewandten Forschung e.V., 80686 München (DE)
(72) Inventor: Muth, Jost, 50969 Köln (DE); Freund, Lena Julie, 52064 Aachen (DE); Edgü, Güven, 52068 Aachen (DE)
(74) Representative: Kuttenkeuler, David

(57) **Abstract**

The invention pertains to methods and means for a specific and fast detection of corona virus nucleic acids in biological samples. The invention provides novel oligonucleotides for use in Reverse Transcription Loop-Mediated Isothermal Amplification (RT-LAMP), which are shown to allow a fast and specific amplification and detection of viral nucleic acids without time consuming sample preparation and purification.

## Description

### FIELD OF THE INVENTION

The invention pertains to methods and means for a specific and fast detection of corona virus nucleic acids in biological samples. The invention provides novel oligonucleotides for use in Reverse Transcription Loop-Mediated Isothermal Amplification (RT-LAMP), which are shown to allow a fast and specific amplification and detection of viral nucleic acids without time consuming sample preparation and purification.

### BACKGROUND

Severe acute respiratory syndrome coronavirus 2 (SARS-CoV-2), a novel coronavirus, has been rapidly spreading around the globe since the beginning of 2020. In people, it causes coronavirus disease 2019 (COVID-19) often accompanied by severe respiratory syndrome (Chen et al., 2020; Zhao et al., 2020; Zhu et al., 2020). To conquer the global health crisis triggered by COVID-19, fast and specific diagnostics are essential. If an infection with the pandemic SARS-CoV-2 is suspected, the pathogen is preferably detected at the nucleic acid level using a standardized molecular biological test for detecting the RNA viral genome, eg by Reverse Transcriptase Polymerase Chain Reaction (RT-PCR). For this purpose, the viral RNA from patient samples (for example from swabs or sputum) is first prepared in the laboratory by a labour-intensive purification process (2-3 hours) before the actual virus detection by RT-PCR using state-of-the-art thermal cyclers can take place. Under ideal conditions such a test takes 3-5 hours in a clinical laboratory environment. In the current pandemic scenario assay time is a huge bottle neck for extensive population wide screening.

An alternative PCR-based method for the detection of pathogens is Reverse Transcription Loop-Mediated Isothermal Amplification (RT-LAMP), which allows the detection of nucleic acids of any origin within a short time (approx. 60 minutes) and does not require the use of complex analytical equipment. Despite their many advantages, RT-LAMPs have not yet found their way into standard clinical diagnostics of human pathogens such as SARS-CoV-2. This is due to the unacceptable abundance of false-positive and false-negative results. This is mainly due to the LAMP probes used (hereinafter also referred to as detection oligonucleotides or primers), whose design is much more complex and demanding in direct comparison to classical PCR (see figure 1). Instead of using two single, amplicon-bound sense and anti-sense primers, the LAMP requires 4-6 probes, each of which binds specifically to 6-8 individual RNA/DNA target regions.

Although only highly conserved genome segments are subject to the probe design, the high number of target oligonucleotides used inevitably increases the probability of mismatches and the formation of artificial primer dimers. The two common inner LAMP probes, the so-called FIP and BIP primers, are typically between 35-45 nucleotides (nt) long and also consist of sense and anti-sense subunits. In the literature it is extensively described that these two probes, which in theory form the loops, are particularly susceptible to mismatching and the collateral occurrence of hairpin structures. Stable hairpins can cause FIPs and BIPs to "clump" and take an inactive form, or, more importantly, the hairpin structure has a so-called 3' complementarity, which can lead to a self-amplifying structure and thus to a false positive result.

In addition, the use of so many (and sometimes very long) primer sequences increases the risk of so-called "sporadic amplification" in relation to exponential amplification in negative control samples. Complex interactions between multiple probes are cited in the relevant literature as an explanation for this. False-negative results are thus mainly caused by an inadequate design of the LAMP primers.

Thus, it is an objection of the invention to provide RT-LAMP oligonucleotides for the detection of corona virus nucleic acids in a sample which overcome the many draw-backs known for RT-LAMP probes.

### BRIEF DESCRIPTION OF THE INVENTION

Generally, and by way of brief description, the main aspects of the present invention can be described as follows:

In **a first aspect,** the invention pertains to a method of detecting presence of a corona virus nucleic acid in a sample, comprising:
- Contacting the sample with at least one set of reverse transcription-loop mediated isothermal amplification (RT-LAMP) primers specific for a corona virus nucleic acid under conditions sufficient for amplification of the corona virus nucleic acid, thereby producing a corona virus nucleic acid amplification product; and
- Detecting the corona virus nucleic acid amplification product, thereby detecting presence of corona virus nucleic acid in the sample.

In **a second aspect,** the invention pertains to an isolated nucleic acid primer, comprising a nucleic acid sequence of any one of SEQ ID NOs: 1 to 73, 94, or 95, or comprising a nucleic acid sequence having at least 80%, preferably 90%, sequence identity to any one of SEQ ID NO: 1 to 73, 94, or 95, or comprising a sequence that hybridizes under stringent conditions to a target nucleic acid comprising a sequence which is complementary to any of SEQ ID NO: 1 to 73, 94, or 95.

In **a third aspect,** the invention pertains to a method of diagnosing a corona virus infection in a subject, the method comprising detecting the presence of a corona virus nucleic acid in a biological sample previously obtained from the subject with a method of the present invention, wherein the presence of the corona virus nucleic acid in the biological sample indicates a corona virus infection in the subject.

In **a fourth aspect,** the invention pertains to a diagnostic kit for use in the detection of a corona virus nucleic acid, comprising one or more of the isolated nucleic acid primers of the second aspect.

In **a fifth aspect,** the invention pertains to a use of the isolated nucleic acid primer of the third aspect or the diagnostic kit of the second aspect in a method of detecting corona virus nucleic acids, preferably according to the first or second aspect.

### DETAILED DESCRIPTION OF THE INVENTION

In the following, the elements of the invention will be described. These elements are listed with specific embodiments; however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

### Definitions

Unless otherwise noted, technical terms are used according to conventional usage.

All publications, patent applications, patents, and other references mentioned herein are incorporated by reference in their entirety for all purposes. All sequences associated with the GenBank Accession Nos. mentioned herein are incorporated by reference in their entirety as were present on February 23, 2021, to the extent permissible by applicable rules and/or law. In the context of the present invention, the following reference of the SARS-CoV2 genome was used: https://www.ncbi.nlm.nih.gov/nuccore/1798174254/. In case of conflict, the present specification, including explanations of terms, will control.

Although methods and materials similar or equivalent to those described herein can be used to practice or test the disclosed technology, suitable methods and materials are described below. The materials, methods, and examples are illustrative only and not intended to be limiting.

In order to facilitate review of the various embodiments of this disclosure, the following explanations of specific terms are provided:

The term "amplification" pertains to increasing the number of copies of a nucleic acid molecule, such as a gene or fragment of a gene, for example at least a portion of a SARS-CoV-2 nucleic acid molecule. The products of an amplification reaction are called amplification products or amplicons. An example of in vitro amplification is the polymerase chain reaction (PCR), in which a sample (such as a biological sample from a subject) is contacted with a pair or set of oligonucleotide primers, under conditions that allow for hybridization of the primers to a nucleic acid molecule in the sample. The primers are extended under suitable conditions, dissociated from the template, and then re-annealed, extended, and dissociated to amplify the number of copies of the nucleic acid molecule. Other examples of in vitro amplification techniques include real-time PCR, quantitative real-time PCR (qPCR), reverse transcription PCR (RT-PCR), quantitative RT-PCR (qRT-PCR), loop-mediated isothermal amplification (LAMP; see Notomi et ah, Nucl. Acids Res. 28:e63, 2000); reverse-transcription LAMP (RT-LAMP); strand displacement amplification (see USPN 5,744,311); transcription- mediated amplification (USPN 5,399,491) transcription-free isothermal amplification (see USPN 6,033,881); repair chain reaction amplification (see WO 90/01069); ligase chain reaction amplification (see EP-A-320 308); gap filling ligase chain reaction amplification (see USPN 5,427,930); coupled ligase detection and PCR (see USPN 6,027,889); and NASBA^{™} RNA transcription-free amplification (see USPN 6,025,134). A preferred amplification is an isothermal amplification.

The term "amplification product or amplicon" refers to specific DNA fragments generated from any primer-directed amplification reaction, in particular an amplification using primer of the invention. The term "target amplification product" or "amplified target" refers to double stranded DNA ("dsDNA") that is generated from a specific target nucleic acid to be identified and quantified by means of primer directed amplification. Such target is preferably a corona virus nucleic acid. Target amplification products may be produced by LAMP, in particular using the nucleic acid primers of the invention. Further, it is understood that all methods of preparing suitable target amplification products are sufficiently controlled so as to eliminate spurious non-target amplification artefacts such as primer-dimer fragments, triple helices and the like. Target amplification products will generally be dsDNA and will be amenable to being bound by intercalating agents.

The term "conditions sufficient for" pertains to any environment that permits the desired activity, for example, that permits specific binding or hybridization between two nucleic acid molecules or that permits reverse transcription and/or amplification of a nucleic acid. Such an environment may include, but is not limited to, particular incubation conditions (such as time and or temperature) or presence and/or concentration of particular factors, for example in a solution (such as buffer(s), salt(s), metal ion(s), detergent(s), nucleotide(s), enzyme(s), and so on).

The term "contact" in context of the present invention means a placement in direct physical association; for example, in solid and/or liquid form. For example, contacting can occur in vitro with one or more primers and/or probes and a biological sample (such as a sample including nucleic acids) in solution.

The term "detectable label" refers to a compound or composition that is conjugated directly or indirectly to another molecule (such as a nucleic acid molecule) to facilitate detection of that molecule. Specific non-limiting examples of labels include fluorescent and fluorogenic moieties (e.g., fluorophores), chromogenic moieties, haptens (such as biotin, digoxigenin, and fluorescein), affinity tags, and radioactive isotopes (such as 32P, 33P, 35S, and 125I). The label can be directly detectable (e.g., optically detectable) or indirectly detectable (for example, via interaction with one or more additional molecules that are in turn detectable). Methods for labeling nucleic acids, and guidance in the choice of labels useful for various purposes, are discussed, e.g., in Sambrook and Russell, in Molecular Cloning: A Laboratory Manual, 3rd Ed., Cold Spring Harbor Laboratory Press (2001) and Ausubel et ah, in Current Protocols in Molecular Biology, Greene Publishing Associates and Wiley-Intersciences (1987, and including updates). Fluorophore: A chemical compound, which when excited by exposure to a particular stimulus, such as a defined wavelength of light, emits light (fluoresces), for example at a different wavelength (such as a longer wavelength of light). Fluorophores are part of the larger class of luminescent compounds. Luminescent compounds include chemiluminescent molecules, which do not require a particular wavelength of light to luminesce, but rather use a chemical source of energy. Therefore, the use of chemiluminescent molecules (such as aequorin) eliminates the need for an external source of electromagnetic radiation, such as a laser.

Examples of particular fluorophores that can be used in the probes and primers disclosed herein are known to those of skill in the art and include 4-acetamido-4'-isothiocyanatostilbene-2,2'disulfonic acid; acridine and derivatives such as acridine and acridine isothiocyanate, 5-(2'-aminoethyl)aminonaphthalene-1-sulfonic acid (EDANS), 4-amino-N-[3-vinylsulfonyl)phenyl]naphthalimide-3,5 disulfonate (Lucifer Yellow VS), N-(4-anilino-1-naphthyl)maleimide, anthranilamide; Brilliant Yellow; coumarin and derivatives such as coumarin, 7-amino-4-methylcoumarin (AMC, Coumarin 120), 7-amino-4-trifluoromethylcouluarin (Coumaran 151); cyanosine; 4',6-diaminidino-2-phenylindole (DAPI); 5',5"-dibromopyrogallol- sulfonephthalein (Bromopyrogallol Red); 7-diethylamino-3-(4'-isothiocyanatophenyl)-4- methylcoumarin; diethylenetriamine pentaacetate; 4,4'-diisothiocyanatodihydro-stilbene-2,2'- disulfonic acid; 4,4'-diisothiocyanatostilbene-2,2'-disulfonic acid; 5-[dimethylamino]naphthalene- 1-sulfonyl chloride (DNS, dansyl chloride); 4-dimethylaminophenylazophenyl-4'-isothiocyanate (DABITC); eosin and derivatives such as eosin isothiocyanate; erythrosin and derivatives such as erythrosin B and erythrosin isothiocyanate; ethidium; fluorescein and derivatives such as 5- carboxyfluorescein (FAM), 5-(4,6-dichlorotriazin-2-yl)aminofluorescein (DTAF), 2'7'-dimethoxy- 4'5'-dichloro-6-carboxyfluorescein (JOE), fluorescein, fluorescein isothiocyanate (FITC), QFITC (XRITC), 6-carboxy-fluorescein (HEX), and TET (tetramethyl fluorescein); fluorescamine; IR144; IR1446; Malachite Green isothiocyanate; 4-methylumbelliferone; ortho-cresolphthalein; nitrotyrosine; pararosaniline; Phenol Red; B-phycoerythrin; o-phthaldialdehyde; pyrene and derivatives such as pyrene, pyrene butyrate, and succinimidyl 1 -pyrene butyrate; Reactive Red 4 (CIBACRON^{™} Brilliant Red 3B-A); rhodamine and derivatives such as 6-carboxy-X-rhodamine (ROX), 6-carboxyrhodamine (R6G), lissamine rhodamine B sulfonyl chloride, rhodamine (Rhod), rhodamine B, rhodamine 123, rhodamine X isothiocyanate, N,N,N',N'-tetramethyl-6-carboxyrhodamine (TAMRA), tetramethyl rhodamine, and tetramethyl rhodamine isothiocyanate (TRITC); sulforhodamine B; sulforhodamine 101 and sulfonyl chloride derivative of sulforhodamine 101 (Texas Red); riboflavin; rosolic acid and terbium chelate derivatives; LightCycler Red 640; Cy5.5; and Cy56-carboxyfluorescein; boron dipyrromethene difluoride (BODIPY); acridine; stilbene; Cy3; Cy5, VIC^{®} (Applied Biosystems); LC Red 640; LC Red 705; and Yakima yellow amongst others. Additional examples of fluorophores include Quasar^{®} 670, Quasar^{®} 570, CalRed 590, CalRed 610, CalRed615, CalRed 635, CalGreen 520, CalGold 540, and CalOrange 560 (Biosearch Technologies, Novato, CA). One skilled in the art can select additional fluorophores, for example those available from Molecular Probes/Life Technologies (Carlsbad, CA).

In particular examples, a fluorophore is used as a donor fluorophore or as an acceptor fluorophore. "Acceptor fluorophores" are fluorophores which absorb energy from a donor fluorophore, for example in the range of about 400 to 900 nm (such as in the range of about 500 to 800 nm). Acceptor fluorophores generally absorb light at a wavelength which is usually at least 10 nm higher (such as at least 20 nm higher) than the maximum absorbance wavelength of the donor fluorophore, and have a fluorescence emission maximum at a wavelength ranging from about 400 to 900 nm. Acceptor fluorophores have an excitation spectrum that overlaps with the emission of the donor fluorophore, such that energy emitted by the donor can excite the acceptor. Ideally, an acceptor fluorophore is capable of being attached to a nucleic acid molecule.

In a particular example, an acceptor fluorophore is a dark quencher, such as Dabcyl, QSY7 (Molecular Probes), QSY33 (Molecular Probes), BLACK HOLE QUENCHERS^{™} (Biosearch Technologies; such as BHQ0, BHQ1, BHQ2, and BHQ3), ECLIPSE^{™} Dark Quencher (Epoch Biosciences), or IOWA BLACK^{™} (Integrated DNA Technologies). A quencher can reduce or quench the emission of a donor fluorophore.

"Donor fluorophores " are fluorophores or luminescent molecules capable of transferring energy to an acceptor fluorophore, in some examples generating a detectable fluorescent signal from the acceptor. Donor fluorophores are generally compounds that absorb in the range of about 300 to 900 nm, for example about 350 to 800 nm. Donor fluorophores have a strong molar absorbance coefficient at the desired excitation wavelength, for example greater than about 103 M"i cm"i.

The term "isolated", e.g. in context of an "isolated" biological component (such as a nucleic acid, or isolated primer) has been substantially separated or purified away from other biological components in which the component naturally occurs, such as other chromosomal and extrachromosomal DNA, RNA, and proteins. Nucleic acids that have been "isolated" include nucleic acids purified by standard purification methods. The term also embraces nucleic acids prepared by recombinant expression in a host cell as well as chemically synthesized nucleic acid molecules. Isolated does not require absolute purity, and can include protein, peptide, or nucleic acid molecules that are at least 50% isolated, such as at least 75%, 80%, 90%, 95%, 98%, 99%, or even 99.9% isolated.

The term "loop-mediated isothermal amplification" or "LAMP" or "RT-LAMP" refers to a method for amplifying DNA or RNA. The method is a single-step amplification reaction utilizing a DNA polymerase with strand displacement activity (e.g., Notomi et ah, Nucl. Acids. Res. 28:E63, 2000; Nagamine et ah, Mol. Cell. Probes 16:223-229, 2002; Mori et al, J. Biochem. Biophys. Methods 59: 145-157, 2004). At least four primers, preferably six primers, which are specific for six to eight regions within a target nucleic acid sequence, are typically used for LAMP. The primers include a forward outer primer (F₃), a backward outer primer (B₃), a forward inner primer (FIP), and a backward inner primer (BIP). A forward loop primer (Loop F), and a backward loop primer (Loop B) can also be included in some embodiments. The amplification reaction produces a stem-loop DNA with inverted repeats of the target nucleic acid sequence. Reverse transcriptase can be added to the reaction for amplification of RNA target sequences. This variation is referred to as RT-LAMP.

A "primer set" according to the invention is a collection of single primers which are in combination sufficient for mediating a LAMP or RT-LAMP.

The term "primer" denotes short nucleic acids, generally DNA oligonucleotides of 10 nucleotides or more in length (such as 10-60, 15-50, 20-45, or 20-40 nucleotides in length). Primers may be annealed to a complementary target DNA strand by nucleic acid hybridization to form a hybrid between the primer and the target DNA strand, and then extended along the target DNA strand by a DNA polymerase enzyme. Primer pairs can be used for amplification of a nucleic acid sequence, e.g., by the polymerase chain reaction (PCR), LAMP, RT-LAMP, or other nucleic acid amplification methods known in the art.

The term "probe" typically refers to an isolated nucleic acid (for example, at least 10 or more nucleotides in length) with an attached detectable label or reporter molecule. Typical labels include radioactive isotopes, ligands, chemiluminescent agents, fluorophores, and enzymes. Methods for labeling oligonucleotides and guidance in the choice of labels appropriate for various purposes are discussed, e.g., in Sambrook et al. (2001) and Ausubel et al. (1987).

The term "recombinant nucleic acid" refers to a nucleic acid molecule that is not naturally occurring or has a sequence that is made by an artificial combination of two otherwise separated segments of nucleotide sequence. This artificial combination is accomplished by chemical synthesis or by the artificial manipulation of isolated segments of nucleic acids, e.g., by genetic engineering techniques such as those described in Sambrook and Russell, in Molecular Cloning: A Laboratory Manual, 3rd Ed., Cold Spring Harbor Laboratory Press (2001). The term "recombinant" includes nucleic acids that have been altered solely by addition, substitution, or deletion of a portion of a natural nucleic acid molecule. A recombinant nucleic acid also includes a heterologous nucleic acid that is inserted in a vector. A "heterologous nucleic acid" refers to a nucleic acid that originates from a different genetic source or species, for example a viral nucleic acid inserted in a bacterial plasmid (referred to herein in some examples as a recombinant vector).

A "sample" (or a "biological sample") is a biological specimen containing DNA (for example, genomic DNA or cDNA), RNA (including mRNA), protein, or combinations thereof. Examples include, but are not limited to isolated nucleic acids, cells, cell lysates, chromosomal preparations, peripheral blood, urine, stool, saliva, eye fluid, tissue biopsy (such as a tumor biopsy or lymph node biopsy), surgical specimen, bone marrow, amniocentesis samples, and autopsy material. In one example, a sample includes viral nucleic acids, for example, SARS-CoV-2 RNA or DNA reverse transcribed from SARS-CoV-2 RNA. In particular examples, samples are used directly (e.g., fresh or frozen), or can be manipulated prior to use, for example, by fixation (e.g., using formalin), addition of RNA protection reagents (https://international.neb.com/products/t2011-monarch-dna-rna-protection-reagent#Product%20Information) and/or embedding in wax (such as FFPE tissue samples). A preferred sample in accordance with the invention is a sample obtained from the respiratory tract of a subject, such as a gurgle sample, a mouth swap sample, a nose swap sample or a throat swap sample.

The term "subject" denotes any multi-cellular vertebrate organism, such as human and non-human mammals (including non-human primates). In one example, a subject is known to be or is suspected of being infected with SARS-CoV-2.

The term "hybridization under stringent conditions" means hybridization under experimental conditions well known to those skilled in the art, for example. Specifically, the term "stringent conditions" refers to conditions such that a nucleic acid hybrid formed is identified after carrying out hybridization at 60°C to 68°C in the presence of 0.7 to 1 mol/l NaCl and then carrying out washing at 65°C to 68°C using a 0.1- to 2-fold SSC solution. Note here that 1×SSC is composed of 150 mmol/l NaCl and 15 mmol/l sodium citrate, for example. In order to select the stringency, for example, the salt concentration and the temperature in the washing step can be optimized as appropriate. Furthermore, it is common general technical knowledge in the art to add, for example, formamide, SDS, or the like to improve the stringency. In some preferred embodiments, stringent hybridization conditions may be those used in a classical LAMP amplification reaction as disclosed herein in the example section.

The term "corona virus nucleic acid" shall pertain to a nucleic acid that is directly derived from a corona virus genome, such as corona viral genomic RNA or, or DNA which was reverse transcribed therefrom. A corona virus in context of the invention is a virus of the *Coronaviridae* family. *Coronaviridae* includes, e.g., coronaviruses, e.g., human coronavirus 229E (HCoV-229E), human coronavirus OC₄₃ (HCoV-OC₄₃), and SARS-CoV and SARS-CoV-2 (the causative agent of severe acute respiratory syndrome (SARS)), which cause upper respiratory tract infection, lower respiratory tract infections, and gastroenteritis. Most preferably in all aspects and embodiments of the herein disclosed invention the corona virus is SARS-CoV-2, the causative virus of the COVID-19 pandemic.

The term "set of primer" or "set of RT-LAMP primer" or similar terms referring to a certain "set" of oligonucleotides shall denote a multiplicity of primer oligonucleotides that together in an amplification reaction can be used to amplify a nucleic acid. Hence, when referring to a "set of RT-LAMP primer", or a "LAMP set" such set requires at least 4 RT-LAMP probes for a sufficient amplification at isothermal conditions according to the general LAMP or RT-LAMP procedure. Such set therefore at least includes the inner primers (forward inner primer, or FIP, and backward inner primer, or BIP) as well the outer primers F₃ and B₃ (see also figure 1). In certain preferred embodiments of the invention a "set of RT-LAMP primer" may additionally comprise forward and backward loop primer (Loop F and Loop B or LF and BL).

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

### Methods, Means and primer sets for Detecting SARS-CoV-2 Nucleic Acids

Disclosed herein are methods and means of detecting SARS-CoV-2 nucleic acids in a sample (such as in a sample from a subject infected with or suspected to be infected with SARS-CoV-2). The disclosed methods can be used to diagnose an infection with SARS-CoV-2 in a subject, for example, by analyzing a biological sample from a subject to detect SARS-CoV-2 nucleic acids in the sample from the subject. In some examples, the methods include LAMP or RT-LAMP assays.

Accordingly, in **a first aspect,** the invention pertains to a method of detecting a presence of a corona virus nucleic acid in a sample, comprising:
- Contacting the sample with at least one set of reverse transcription-loop mediated isothermal amplification (RT-LAMP) primers specific for a corona virus nucleic acid under conditions sufficient for amplification of the corona virus nucleic acid, thereby producing a corona virus nucleic acid amplification product; and
- Detecting the corona virus nucleic acid amplification product, thereby detecting presence of corona virus nucleic acid in the sample.

In preferred embodiments of the invention the corona virus of which a nucleic acid is detected with the method and means of the invention is SARS-CoV-2. In certain particular preferred embodiments of the invention, the methods and means of the various aspects and embodiments disclosed herein, relate to a specific and selective detection of SARS-CoV-2, and thus, they specifically exclude, or the methods and means of the invention cannot detect, other corona viruses, preferably not human coronavirus 229E (HCoV- 229E) and human coronavirus OC₄₃ (HCoV-OC₄₃).

The methods and means described herein may be used for any purpose for which detection of SARS-CoV-2 nucleic acids is desirable, including diagnostic and prognostic applications, such as in laboratory and clinical settings. Appropriate samples include any conventional biological samples, including clinical samples obtained from a human or veterinary subject. Suitable samples include all biological samples useful for detection of infection in subjects, including, but not limited to, cells (such as buccal cells or peripheral blood mononuclear cells), tissues, autopsy samples, bone marrow aspirates, bodily fluids (for example, blood, serum, plasma, urine, stool, cerebrospinal fluid, middle ear fluids, eye fluids, breast milk, bronchoalveolar lavage, tracheal aspirates, sputum, oral fluids, nasopharyngeal aspirates, oropharyngeal aspirates, or saliva), oral swabs, eye swabs, cervical swabs, vaginal swabs, rectal swabs, stool, and stool suspensions. In the context of the present invention, preferred samples are selected from throat swab, nasal swab, oral swab, swabs from mucous membranes, and gargle water. The sample can be used directly, which is one specific advantage of the herein disclosed methods for detection of SARS-CoV-2, or can be processed, such as by adding solvents, preservatives, buffers, or other compounds or substances. In some examples, nucleic acids are isolated from the sample. In other examples, isolation of nucleic acids from the sample is not necessary prior to use in the methods disclosed herein and the sample (such as a saliva or gargle) is used directly or with minimal pre-processing. For example, bodily fluids (such as a saliva/sputum or gargle) can in some examples be diluted in water or buffer and used in the disclosed RT-LAMP assays without additional processing. In some examples, the sample (for example, a sample containing cells, such as peripheral blood mononuclear cells) is pre-treated to lyse cells (for example with a lysis buffer) or by simple heating, but nucleic acids are not isolated prior to use in the RT-LAMP assay. Samples also include isolated nucleic acids, such as DNA or RNA isolated from a biological specimen from a subject, a SARS-CoV-2 isolate, or other source of nucleic acids. Methods for extracting nucleic acids such as RNA and/or DNA from a sample are known to one of skill in the art; such methods will depend upon, for example, the type of sample in which the nucleic acid is found. Nucleic acids can be extracted using standard methods. For instance, rapid nucleic acid preparation can be performed using a commercially available kit (such as kits and/or instruments from Qiagen (such as DNEasy^{®} or RNEasy^{®} kits), Roche Applied Science (such as MagNA Pure kits and instruments), Thermo Scientific (KingFisher mL), bioMerieux (Nuclisens^{®} NASBA Diagnostics), or Epicentre (Masterpure^{™} kits)). In other examples, the nucleic acids may be extracted using guanidinium isothiocyanate, such as single-step isolation by acid guanidinium isothiocyanate-phenolchloroform extraction (Chomczynski et al. Anal. Biochem. 162: 156-159, 1987).

One additional advantage of the methods and means of the present invention is compared to standard RT-PCR based methodologies is the significantly reduced assay and detection time, preferably up to 45 minutes, while increasing amplification rate and thus enhancing the detection limits of the assay. The probe optimization of the invention provides a highly specific yet fast and cost reduced diagnostic for SARS-CoV-2 infections even working in low viral samples such as gargle. These effects improve testing acceptance and speed. These effects are possible for the optimized primer sequences of the invention.

Disclosed herein are methods for detecting SARS-CoV-2 in a sample utilizing LAMP or RT- LAMP methods of amplification and detection. LAMP, which was first described by Notomi et al. (Nucl. Acids Res. 28:e63, 2000), is a one-step isothermal amplification method that can produce amplified nucleic acids in a short period of time using a DNA polymerase with strand displacement activity. LAMP can be adapted for amplification of RNA targets with the addition of reverse transcriptase (RT) to the reaction without an additional heat step (referred to as RT-LAMP). The isothermal nature of LAMP and RT-LAMP allows for assay flexibility because it can be used with simple and inexpensive heating devices, which can facilitate SARS-CoV-2 detection in settings other than centralized clinical laboratories, including at the point-of-care (POC). POC testing is particularly important for SARS-CoV-2 diagnosis, as it has the potential to allow decentralized population wide screening of SARS-CoV-2 infections. In addition, LAMP and RT-LAMP offer versatility in terms of specimen type and is believed to increase the probability of detecting an amplifiable target even in gargle, which will increase general testing acceptance.

In preferred embodiments of the herein disclosed invention the at least one set of RT-LAMP primers specific for the corona virus nucleic acid comprises one or more of a forward outer primer (F3), a backward outer primer (B3), a forward inner primer (FIP), a backward inner primer (BIP), and preferably: a forward loop primer (FL), and a backward loop primer (BL).

In some embodiments of the invention it is preferred that the at least one set of RT-LAMP primers is specific for a region within the corona virus genome selected from: a region within the corona virus gene ORF1AB, or a region within the corona virus gene N, preferably wherein the region within the corona virus gene ORF1AB is at a genomic location from about 500 to about 800; and wherein the region within the gene N fragment is at a genomic location from about 28500 to about 28900. The genome of SARS-CoV-2 is known in the art (https://www.ncbi.nlm.nih.gov/nuccore/1798174254/).

The present invention in particular pertains to a set of nucleic acid primers for use in Loop-Mediated Isothermal Amplification (LAMP) of a Severe Acute Respiratory Syndrome-Corona Virus 2 (SARS-Cov2) derived target nucleic acid, comprising at least a first Corona LAMP Primer Set, wherein the first Corona LAMP Primer Set comprises at least the following nucleic acid primer molecules:
- A nucleic acid **F3 LAMP primer** which (i) comprises, or consists essentially of, a sequence shown in SEQ ID NO: 47 (GGACCCCAAAATCAG), or a sequence having no more than 5, or no more than 3, preferably no more than 1 nucleotide alterations therefrom selected from addition(s), deletion(s), and substitution(s), or (ii) hybridizes under stringent conditions to a target nucleic sequence which is complementary to the sequence shown in SEQ ID NO: 47 (GGACCCCAAAATCAG; and
- A nucleic acid **FL LAMP primer** which (i) comprises, or consists essentially of, a sequence shown in SEQ ID NO: 94 (GTTGAATCTGAGGGTCC), or a sequence having no more than 5, or no more than 3, preferably no more than 1 nucleotide alterations therefrom selected from addition(s), deletion(s), and substitution(s), or (ii) hybridizes under stringent conditions to a target nucleic sequence which is complementary to the sequence shown in SEQ ID NO: 94 (GTTGAATCTGAGGGTCC); and
- A nucleic acid **FIP LAMP primer** which (i) comprises, or consists essentially of, a sequence shown in SEQ ID NO: 95 (TCTCCATTCTGGTTACTGCCCGAAATGCACCCCGCATTAC), or a sequence having no more than 5, or no more than 3, preferably no more than 1 nucleotide alterations therefrom selected from addition(s), deletion(s), and substitution(s), or (ii) hybridizes under stringent conditions to a target nucleic sequence which is complementary to the sequence shown in SEQ ID NO: 95 (TCTCCATTCTGGTTACTGCCCGAAATGCACCCCGCATTAC); and
- A nucleic acid **B3 LAMP primer** which (i) comprises, or consists essentially of, a sequence shown in SEQ ID NO: 56 (CTTGCCATGTTGAGTG), or a sequence having no more than 5, or no more than 3, preferably no more than 1 nucleotide alterations therefrom selected from addition(s), deletion(s), and substitution(s), or (ii) hybridizes under stringent conditions to a target nucleic sequence which is complementary to the sequence shown in SEQ ID NO: 56 (CTTGCCATGTTGAGTG); and
- A nucleic acid **BL LAMP primer** which (i) comprises, or consists essentially of, a sequence shown in SEQ ID NO: 53 (CCCCAAGGTTTACCC), or a sequence having no more than 5, or no more than 3, preferably no more than 1 nucleotide alterations therefrom selected from addition(s), deletion(s), and substitution(s), or (ii) hybridizes under stringent conditions to a target nucleic sequence which is complementary to the sequence shown in SEQ ID NO: 53 (CCCCAAGGTTTACCC); and
- A nucleic acid **BIP LAMP primer** which (i) comprises, or consists essentially of, a sequence shown in SEQ ID NO: 55 (CGCGATCAAAACAACGTCGGAGCGGTGAACCAAGACGCAG), or a sequence having no more than 5, or no more than 3, preferably no more than 1 nucleotide alterations therefrom selected from addition(s), deletion(s), and substitution(s), or (ii) hybridizes under stringent conditions to a target nucleic sequence which is complementary to the sequence shown in SEQ ID NO: 55 (CGCGATCAAAACAACGTCGGAGCGGTGAACCAAGACGCAG).

In addition, the first aspect pertains also to a set of nucleic acid primers for use in Loop-Mediated Isothermal Amplification (LAMP) of a Severe Acute Respiratory Syndrome-Corona Virus 2 (SARS-Cov2) derived target nucleic acid, comprising at least a second Corona LAMP Primer Set, wherein the second Corona LAMP Primer Set comprises at least the following nucleic acid primer molecules:
- A nucleic acid **F3 LAMP primer** which (i) comprises, or consists essentially of, a sequence shown in SEQ ID NO: 1 (ATGACCAAATTGGCTACTAC), or a sequence having no more than 5, or no more than 3, preferably no more than 1 nucleotide alterations therefrom selected from addition(s), deletion(s), and substitution(s), or (ii) hybridizes under stringent conditions to a target nucleic sequence which is complementary to the sequence shown in SEQ ID NO: 1 (ATGACCAAATTGGCTACTAC); and
- A nucleic acid **FL LAMP primer** which (i) comprises, or consists essentially of, a sequence shown in SEQ ID NO: 12 (CCATCTTGGACTGAG), or a sequence having no more than 5, or no more than 3, preferably no more than 1 nucleotide alterations therefrom selected from addition(s), deletion(s), and substitution(s), or (ii) hybridizes under stringent conditions to a target nucleic sequence which is complementary to the sequence shown in SEQ ID NO: 12 (CCATCTTGGACTGAG); and
- A nucleic acid **FIP LAMP primer** which (i) comprises, or consists essentially of, a sequence shown in SEQ ID NO: 4 (AGCTTCTGGCCCAGTTCCTTCGTGGTGGTGACGG), or a sequence having no more than 5, or no more than 3, preferably no more than 1 nucleotide alterations therefrom selected from addition(s), deletion(s), and substitution(s), or (ii) hybridizes under stringent conditions to a target nucleic sequence which is complementary to the sequence shown in SEQ ID NO: 4 (AGCTTCTGGCCCAGTTCCTTCGTGGTGGTGACGG); and
- A nucleic acid **B3 LAMP primer** which (i) comprises, or consists essentially of, a sequence shown in SEQ ID NO: 68 (AGCACGATTGCAGCAT), or a sequence having no more than 5, or no more than 3, preferably no more than 1 nucleotide alterations therefrom selected from addition(s), deletion(s), and substitution(s), or (ii) hybridizes under stringent conditions to a target nucleic sequence which is complementary to the sequence shown in SEQ ID NO: 68 (AGCACGATTGCAGCAT); and
- A nucleic acid **BL LAMP primer** which (i) comprises, or consists essentially of, a sequence shown in SEQ ID NO: 65 (ACTGAGGGAGCCTTGA), or a sequence having no more than 5, or no more than 3, preferably no more than 1 nucleotide alterations therefrom selected from addition(s), deletion(s), and substitution(s), or (ii) hybridizes under stringent conditions to a target nucleic sequence which is complementary to the sequence shown in SEQ ID NO: 65 (ACTGAGGGAGCCTTGA); and
- A nucleic acid **BIP LAMP primer** which (i) comprises, or consists essentially of, a sequence shown in SEQ ID NO: 67 (CAAAGACGGCATCATATGGGGGATTGCGGGTGCCAATGTG), or a sequence having no more than 5, or no more than 3, preferably no more than 1 nucleotide alterations therefrom selected from addition(s), deletion(s), and substitution(s), or (ii) hybridizes under stringent conditions to a target nucleic sequence which is complementary to the sequence shown in SEQ ID NO: 67 (CAAAGACGGCATCATATGGGGGATTGCGGGTGCCAATGTG).

The set of nucleic acid primers of the invention may in certain embodiments comprise a duplex or triplex combination of LAMP primer sets. In such duplex or triplex applications each LAMP primer set is associated with a distinct detectable label to allow a specific detection of amplification based on each set independently. Hence, in preferred embodiments of the invention the set of nucleic acid primers comprises both the first Corona LAMP Primer Set recited above and the second Corona LAMP Primer Set recited above.

In a triplex application the set of nucleic acid primers of the invention further comprises a Control LAMP Primer Set, which comprises nucleic acid primers for the LAMP of an internal control reverse transcribed nucleic acid such as a reverse transcribed RNA derived from a housekeeping gene, such as human beta actin (ACTB).

In this context it may be preferred that the F3 LAMP primer, the FL LAMP Primer, B3 LAMP primer, and/or the BL LAMP Primer each have, or consist of, a length of 5 to 30 nucleotides (nt), preferably of 8 to 25 nt.

In this context it may be also preferred that the FIP LAMP primer, and/or the BIP LAMP Primer each have, or consist of, a length of 10 to 50 nucleotides (nt), preferably of 15 to 40 nt.

As mentioned above at least one primer in each LAMP primer set, preferably more than one primer in each of such set, comprises a detectable label, such as a detectable label selected from the group consisting of biotin, FAM, DIG or any other example for detectable labels as described herein elsewhere.

In further preferred embodiments of the invention the at least one set of RT-LAMP primers comprises at least one, preferably two, four or six, primer comprising a nucleic acid sequence at least 80%, preferably 85%, 90%, 95%, 96%, 97%, 98%, 99%, or most preferably 100% identical to any one of SEQ ID NOs: 1 to 73, 94, or 95, or comprising a nucleic sequence that hybridizes under stringent conditions to a target nucleic acid comprising a sequence which is complementary to any one of SEQ ID NO: 1 to 73, 94, or 95.

As mentioned above, LAMP or RT-LAMP can also be multiplexed through the addition of multiple LAMP primer sets with different specificities. This capability is advantageous, for example, because it allows for incorporation of internal control(s), amplification of two or more regions within the same target, or detection of two or more targets or pathogens in a single reaction. In some examples, the disclosed methods include a multiplex LAMP or RT-LAMP assay for detection of multiple regions of SARS-CoV-2 nucleic acids in a single reaction, for example targeting ORF1AB and the N gene.

In other embodiments, the means and methods include contacting a sample (such as a sample including or suspected to include SARS CoV-2 nucleic acids) with at least one set of LAMP primers specific for a SARS CoV-2 ORF1AB nucleic acid (for example, a set of primers including the sequences of SEQ ID NOs: 19-30; and 57-63) under conditions sufficient for amplification of the SARS CoV2 ORF1AB nucleic acid, producing an amplification product. In some examples, the LAMP primers amplify a SARS CoV-2 ORF1AB nucleic acid having at least 80% sequence identity (such as at least 85%, 90%, 95%, 98%, or more sequence identity) to nucleotides 500-800 of the SARS CoV2 reference sequence. In some examples, the methods further include reverse transcription of SARS CoV-2 RNA in the sample, for example by contacting the sample with a reverse transcriptase. Contacting the sample with reverse transcriptase may be prior to contacting the sample with the one or more sets of LAMP primers, or may be simultaneous with contacting the sample with the one or more sets of LAMP primers (for example in the same reaction mix with the LAMP primers). The amplification product is detected by any suitable method, such as detection of turbidity, fluorescence, lateral flow immunochromatographic assays (hereinafter termed as "dipsticks") or by gel electrophoresis.

In some embodiments, the set of LAMP primers specifically amplifies a SARS CoV-2 ORF1AB nucleic acid. An exemplary set of LAMP primers for amplification of an ORF1AB nucleic acid includes an F3 primer including a nucleic acid with at least 90% (such as at least 95%, 96%, 98%, or preferably 100%) sequence identity to SEQ ID NO: 19, a B3 primer including a nucleic acid with at least 90% (such as at least 95%, 96%, 98%, or preferably 100%) sequence identity to SEQ ID NO: 26, an FIP primer including a nucleic acid with at least 90% (such as at least 95%, 96%, 98%, or preferably 100%) sequence identity to SEQ ID NO: 22 or 23, and a BIP primer including a nucleic acid with at least 90% (such as at least 95%, 96%, 98%, or preferably 100%) sequence identity to SEQ ID NO: 27, and optionally including a Loop F primer including a nucleic acid with at least 90% (such as at least 95%, 96%, 98%, or preferably 100%) sequence identity to SEQ ID NO: 28 or 29, and a Loop B primer including a nucleic acid with at least 90% (such as at least 95%, 96%, 98%, or preferably 100%) sequence identity to SEQ ID NO: 30, or the reverse complement of any of SEQ ID NOs: 19-30.

LAMP primers include oligonucleotides between 15 and 60 nucleotides in length. In some embodiments, the set of LAMP primers specifically amplifies a SARS CoV-2 N-gene nucleic acid. An exemplary set of LAMP primers for amplification of an SARS CoV-2 N-gene nucleic acid includes an F3 primer including a nucleic acid with at least 90% (such as at least 95%, 96%, 98%, or preferably 100%) sequence identity to SEQ ID NO: 1, a B3 primer including a nucleic acid with at least 90% (such as at least 95%, 96%, 98%, or preferably 100%) sequence identity to SEQ ID NO: 9, an FIP primer including a nucleic acid with at least 90% (such as at least 95%, 96%, 98%, or preferably 100%) sequence identity to SEQ ID NO: 4, 5 or 6, and a BIP primer including a nucleic acid with at least 90% (such as at least 95%, 96%, 98%, or preferably 100%) sequence identity to SEQ ID NO: 10, 11, or 18 and optionally including a Loop F primer including a nucleic acid with at least 90% (such as at least 95%, 96%, 98%, or preferably 100%) sequence identity to SEQ ID NO: 12, 13, and a Loop B primer including a nucleic acid with at least 90% (such as at least 95%, 96%, 98%, or preferably 100%) sequence identity to SEQ ID NO: 14, or the reverse complement of any of SEQ ID NOs: 1-18.

In further embodiments, the set of LAMP primers specifically amplifies a further SARS CoV-2 ORF1AB nucleic acid. A further exemplary set of LAMP primers for amplification of a further ORF1AB nucleic acid includes an F3 primer including a nucleic acid with at least 90% (such as at least 95%, 96%, 98%, or preferably 100%) sequence identity to SEQ ID NO: 57, a B3 primer including a nucleic acid with at least 90% (such as at least 95%, 96%, 98%, or preferably 100%) sequence identity to SEQ ID NO: 68, an FIP primer including a nucleic acid with at least 90% (such as at least 95%, 96%, 98%, or preferably 100%) sequence identity to SEQ ID NO: 59, and a BIP primer including a nucleic acid with at least 90% (such as at least 95%, 96%, 98%, or preferably 100%) sequence identity to SEQ ID NO: 62, and optionally including a Loop F primer including a nucleic acid with at least 90% (such as at least 95%, 96%, 98%, or preferably 100%) sequence identity to SEQ ID NO: 28 or 29, and a Loop B primer including a nucleic acid with at least 90% (such as at least 95%, 96%, 98%, or preferably 100%) sequence identity to SEQ ID NO: 30, or the reverse complement of any of SEQ ID NOs: 28-30, 57-68.

In further embodiments, the set of LAMP primers specifically amplifies a further SARS CoV-2 N-gene nucleic acid. A further exemplary set of LAMP primers for amplification of an SARS CoV-2 N-gene nucleic acid includes an F3 primer including a nucleic acid with at least 90% (such as at least 95%, 96%, 98%, or preferably 100%) sequence identity to SEQ ID NO: 31,32, or 33, a B3 primer including a nucleic acid with at least 90% (such as at least 95%, 96%, 98%, or preferably 100%) sequence identity to SEQ ID NO: 9, 38, or 39, an FIP primer including a nucleic acid with at least 90% (such as at least 95%, 96%, 98%, or preferably 100%) sequence identity to SEQ ID NO: 36, and a BIP primer including a nucleic acid with at least 90% (such as at least 95%, 96%, 98%, or preferably 100%) sequence identity to SEQ ID NO: 10, or 11, and optionally including a Loop F primer including a nucleic acid with at least 90% (such as at least 95%, 96%, 98%, or preferably 100%) sequence identity to SEQ ID NO: 37, and a Loop B primer including a nucleic acid with at least 90% (such as at least 95%, 96%, 98%, or preferably 100%) sequence identity to SEQ ID NO: 14, or the reverse complement of any of SEQ ID NOs: 9-11, 14, 31-39.

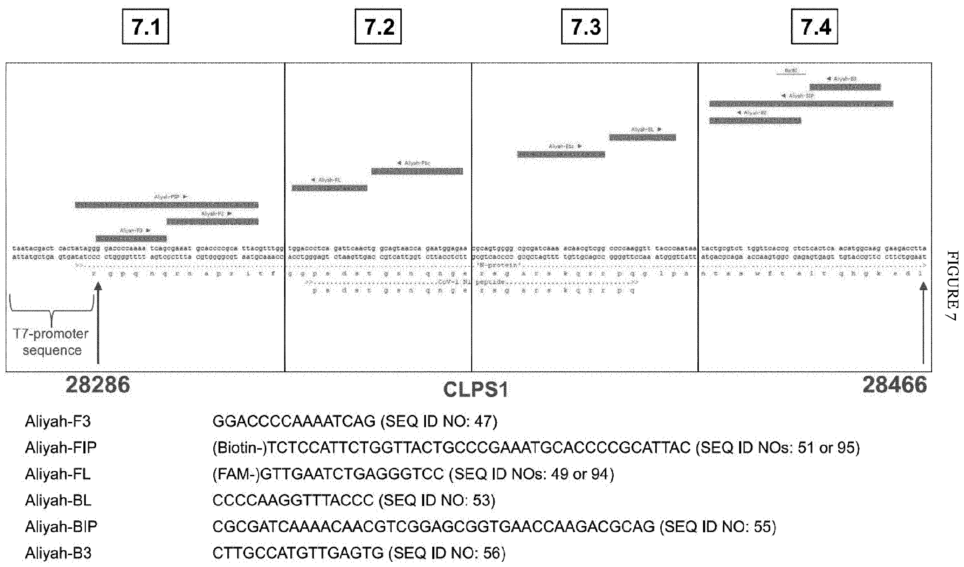

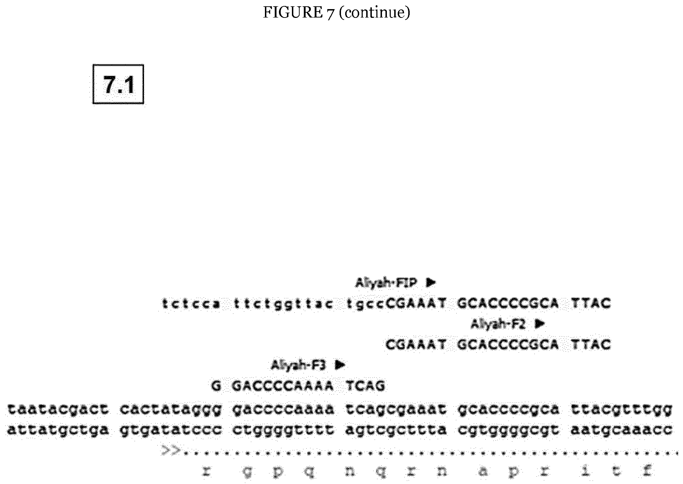

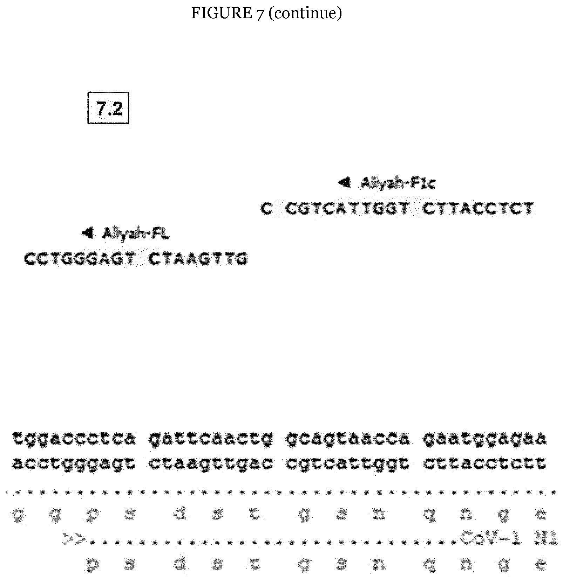

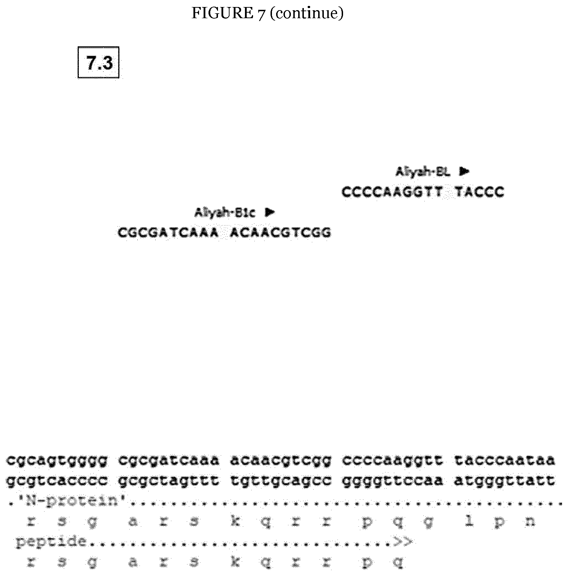

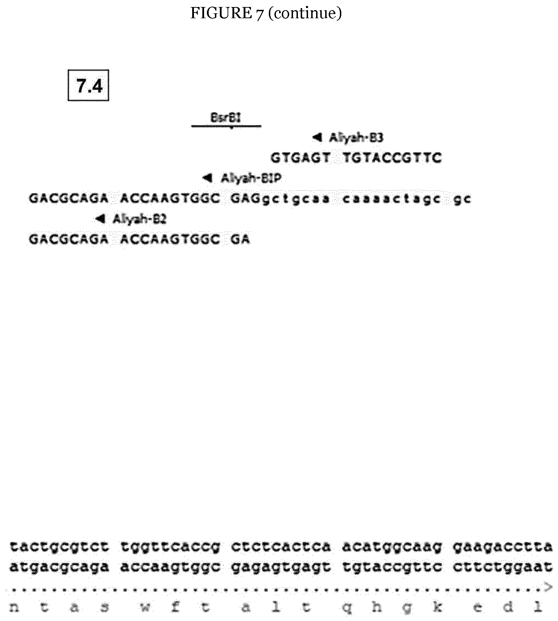

In further embodiments, the set of LAMP primers specifically amplifies a further SARS CoV-2N-gene nucleic acid. A further exemplary set of LAMP primers for amplification of a further SARS CoV-2 N-gene nucleic acid includes an F3 primer including a nucleic acid with at least 90% (such as at least 95%, 96%, 98%, or preferably 100%) sequence identity to SEQ ID NO:47, a B3 primer including a nucleic acid with at least 90% (such as at least 95%, 96%, 98%, or preferably 100%) sequence identity to SEQ ID NO: 56, an FIP primer including a nucleic acid with at least 90% (such as at least 95%, 96%, 98%, or preferably 100%) sequence identity to SEQ ID NO: 51, or 95 and a BIP primer including a nucleic acid with at least 90% (such as at least 95%, 96%, 98%, or preferably 100%) sequence identity to SEQ ID NO: 55, and optionally including a Loop F primer including a nucleic acid with at least 90% (such as at least 95%, 96%, 98%, or preferably 100%) sequence identity to SEQ ID NO: 49, or 94 and a Loop B primer including a nucleic acid with at least 90% (such as at least 95%, 96%, 98%, or preferably 100%) sequence identity to SEQ ID NO: 53, or the reverse complement of any of SEQ ID Nos: 47-56, 94, or 95. This preferred primer set, referred to herein as "CLPSi", is exemplary shown in Figure 7, where the primer binding sites of the primer set CLPS1 in the N gene genomic region of SARS CoV-2 are shown. CLPS1 is further described in Table 1 below.

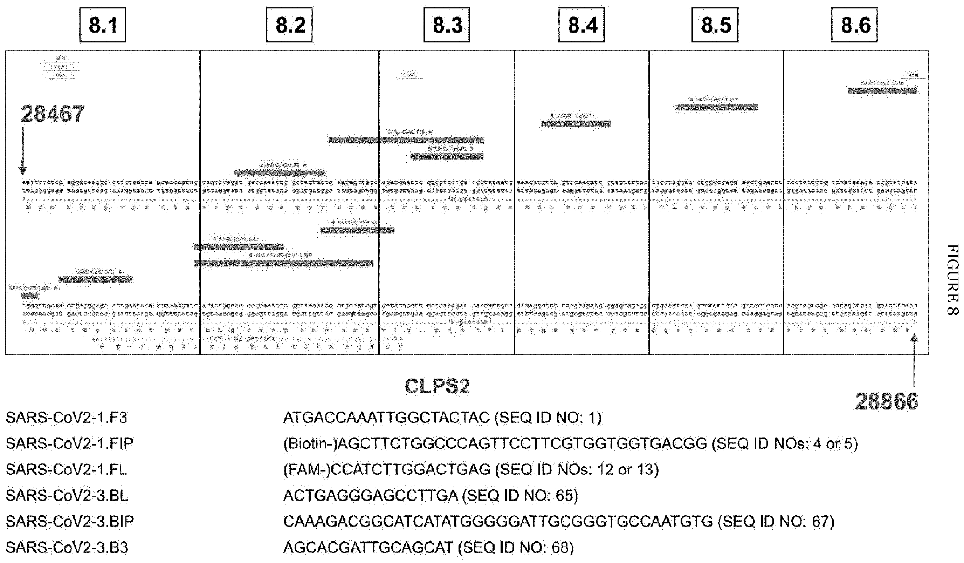

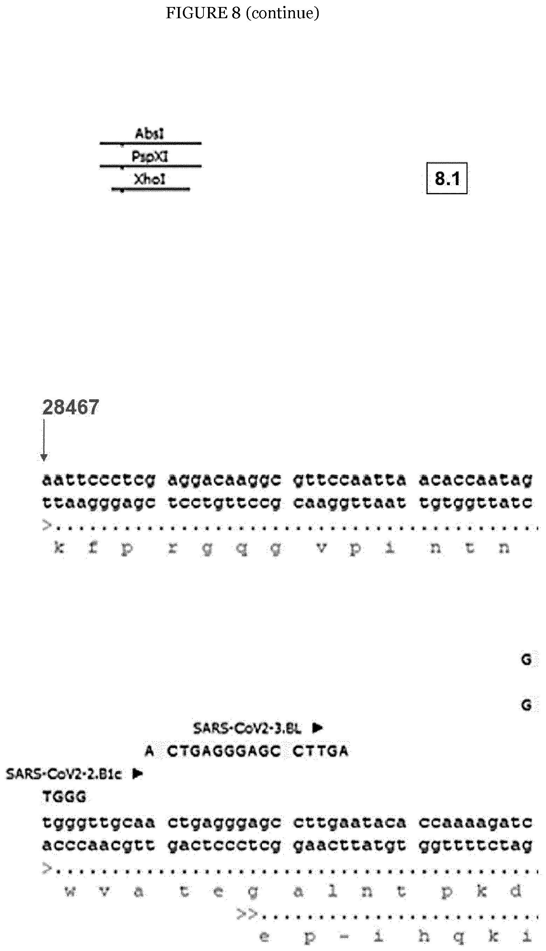

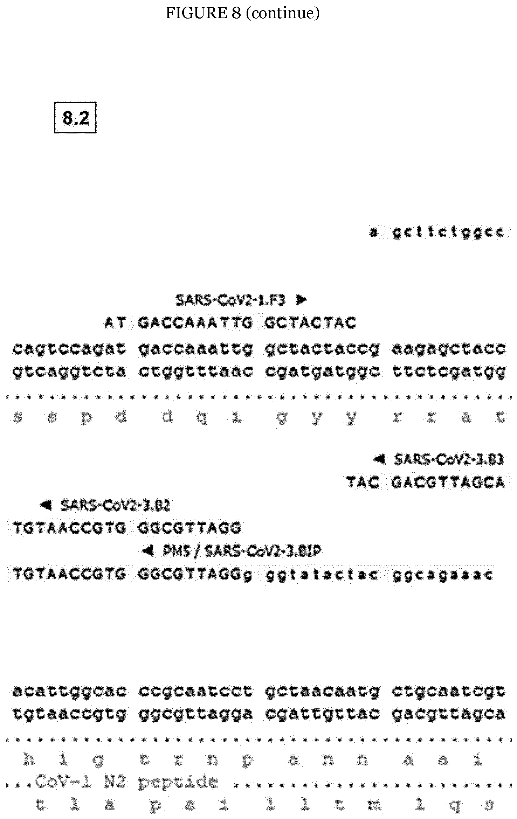

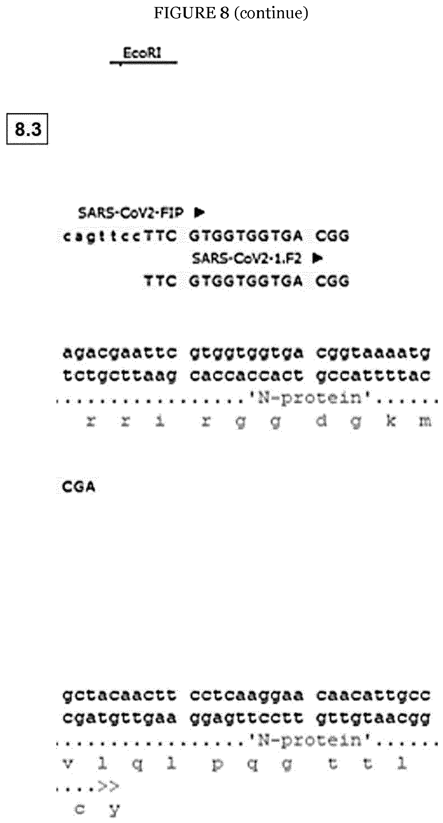

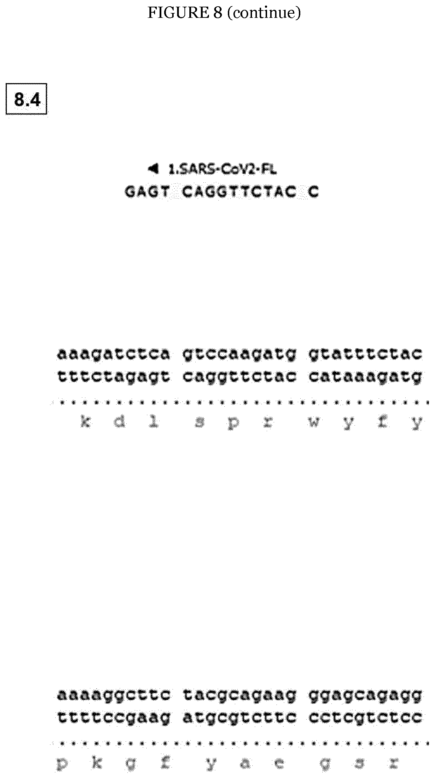

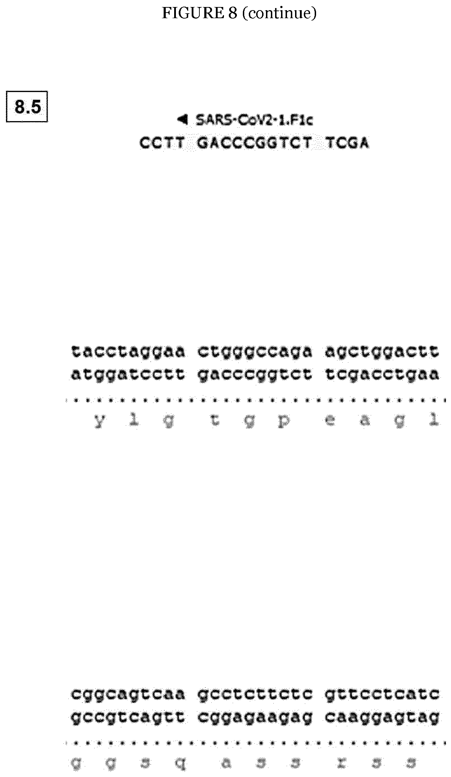

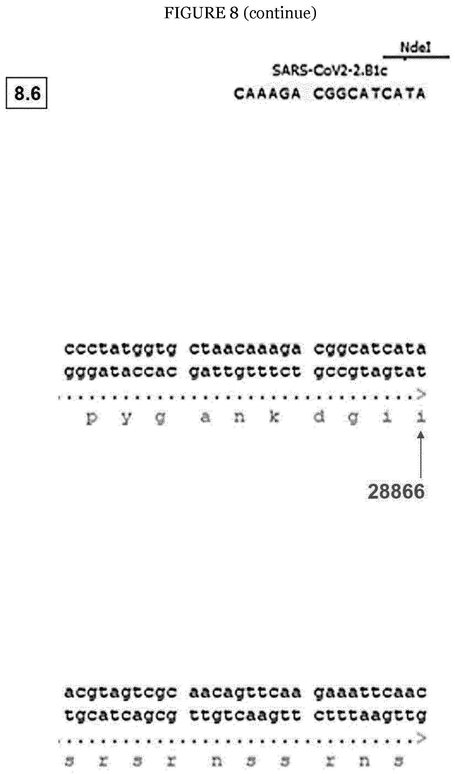

In further embodiments, the set of LAMP primers specifically amplifies a further SARS CoV-2 N-gene nucleic acid. A further exemplary set of LAMP primers for amplification of a further SARS CoV-2 N-gene nucleic acid includes an F3 primer including a nucleic acid with at least 90% (such as at least 95%, 96%, 98%, or preferably 100%) sequence identity to SEQ ID NO: 1, a B3 primer including a nucleic acid with at least 90% (such as at least 95%, 96%, 98%, or preferably 100%) sequence identity to SEQ ID NO:68, an FIP primer including a nucleic acid with at least 90% (such as at least 95%, 96%, 98%, or preferably 100%) sequence identity to SEQ ID NO: 4 or 5, and a BIP primer including a nucleic acid with at least 90% (such as at least 95%, 96%, 98%, or preferably 100%) sequence identity to SEQ ID NO: 67 and optionally including a Loop F primer including a nucleic acid with at least 90% (such as at least 95%, 96%, 98%, or preferably 100%) sequence identity to SEQ ID NO: 12, or 13, and a Loop B primer including a nucleic acid with at least 90% (such as at least 95%, 96%, 98%, or preferably 100%) sequence identity to SEQ ID NO: 65, or the reverse complement of any of SEQ ID NOs: 1, 4, 5, 12, 13, 65, 67, 68. This preferred primer set, referred to herein as "CLPS2", is exemplary shown in Figure 8, where the primer binding sites of the primer set CLPS2 in the N gene genomic region of SARS CoV-2 are shown. CLPS2 is further described in Table 1 below.

Table 1 shows both preferred primer sets CLPS1 and CLPS2 of the present invention with target SARS CoV2 genomic region coordinates.

| **Primer set** | **Primer Designation** | **Sequence** | **Genomic coordinates** | **SEQ ID NO:** | **Suitable label** |
|---|---|---|---|---|---|
| **CLPS1** | Aliyah-F₃ | GGACCCCAAAATCAG | 28286-28300 | 47 | - |
| | Aliyah- FIP | TCTCCATTCTGGTTACTGCCC GAAATGCACCCCGCATTAC | 28365-28346; 28301-28320 | 95 | Biotin (SEQ ID NO: 51) |
| | Aliyah-FL | GTTGAATCTGAGGGTCC | 28344-28328 | 94 | FAM (SEQ ID NO: 49) |
| | Aliyah- BL | CCCCAAGGTTTACCC | 28397-28411 | 53 | - |
| | Aliyah- BIP | CGCGATCAAAACAACGTCGG AGCGGTGAACCAAGACGCAG | 28377-28396; 28438-28419 | 55 | - |
| | Aliyah-B3 | CTTGCCATGTTGAGTG | 28457-28441 | 56 | - |
| **CLPS2** | SARS-CoV2-t.F3 | ATGACCAAATTGGCTACTAC | 28515-28534 | 1 | - |
| | (Biotin-)SARS-CoV2-i.FIP | AGCTTCTGGCCCAGTTCCTTC GTGGTGGTGACGG | 28630-28613; 28554-28569 | 4 | Biotin (SEQ ID NO: 5) |
| | (FAM-)SARS-CoV2-1.FL | CCATCTTGGACTGAG | 28597-28583 | 12 | FAM (SEQ ID NO: 13) |
| | SARS-CoV2-3.BL | ACTGAGGGAGCCTTGA | 28676-28691 | 65 | - |
| | SARS-COV2-3.BIP | CAAAGACGGCATCATATGGG GGATTGCGGGTGCCAATGTG | 28651-28670; 28725-28706 | 67 | - |
| | SARS-CoV2-3.B3 | AGCACGATTGCAGCAT | 28749-28734 | 68 | - |

In further embodiments, the set of LAMP primers specifically amplifies a further SARS CoV-2 N-gene nucleic acid. A further exemplary set of LAMP primers for amplification of a further SARS CoV-2 N-gene nucleic acid includes an F3 primer including a nucleic acid with at least 90% (such as at least 95%, 96%, 98%, or preferably 100%) sequence identity to SEQ ID NO: 40, a B3 primer including a nucleic acid with at least 90% (such as at least 95%, 96%, 98%, or preferably 100%) sequence identity to SEQ ID NO:9, an FIP primer including a nucleic acid with at least 90% (such as at least 95%, 96%, 98%, or preferably 100%) sequence identity to SEQ ID NO: 44 or 45, and a BIP primer including a nucleic acid with at least 90% (such as at least 95%, 96%, 98%, or preferably 100%) sequence identity to SEQ ID NO: 17 and optionally including a Loop F primer including a nucleic acid with at least 90% (such as at least 95%, 96%, 98%, or preferably 100%) sequence identity to SEQ ID NO: 46, and a Loop B primer including a nucleic acid with at least 90% (such as at least 95%, 96%, 98%, or preferably 100%) sequence identity to SEQ ID NO: 14, or 18, or the reverse complement of any of SEQ ID NOs: 9, 14, 17, 18, 40-46.

Further preferred is at least one set of RT-LAMP primers, wherein the at least one set of RT-LAMP primers is specific for a region within the corona virus genome at a genomic location in the range from about 28,200bp to 28,900bp, preferably from about 28,200bp to 28,800bp, or most preferably from about 28,286bp to 28,760bp.

In one preferred embodiment, a set of LAMP primers specifically amplifies a further SARS CoV-2 nucleic acid in the range from about 28,200bp to 28,500bp, preferably from about 28,250bp to 28,480bp, or most preferably from about 28,286bp to 28,457bp of the viral genome of SARS CoV-2, wherein the set of LAMP primers comprises an F3 primer comprising a nucleic acid which binds from about 28,200bp to 28,300bp, preferably from about 28,240bp to 28,300bp and most preferably from about 28,286bp to 28,300bp, such as, for example SEQ ID No: 47; a B3 primer comprising a nucleic acid which binds from about 28,439bp to 28,500bp, preferably from about 28,440bp to 28,480bp and most preferably from about 28,441bp to 28,457bp, such as, for example SEQ ID NO: 56; an FIP primer comprising a nucleic acid which binds from about 28,301bp to 28,371bp, preferably from about 28,301bp to 28,368bp and most preferably from about 28,301bp to 28,365bp, such as, for example SEQ ID NO: 51, or 95; and a BIP primer comprising a nucleic acid which binds from about 28,375bp to 28,440bp, preferably from about 28,376bp to 28,439bp and most preferably from about 28,377bp to 28,438bp, such as, for example SEQ ID NO: 55; and optionally comprising a Loop F primer comprising a nucleic acid which binds from about 28,321bp to 28,346bp, preferably from about 28,324bp to 28,345bp and most preferably from about 28,344bp to 28,328bp, such as, for example SEQ ID NO: 49, or 94; and a Loop B primer comprising a nucleic acid which binds from about 28,397bp to 28,418bp, preferably from about 28,397bp to 28,414bp and most preferably from about 28,397bp to 28,411bp such as, for example SEQ ID NO: 53; of the viral genome of SARS CoV-2.

In a further preferred embodiment, a set of LAMP primers specifically amplifies a further SARS CoV-2 nucleic acid in the range from about 28,500bp to 28,900bp, preferably from about 28,560bp to 28,800bp, or most preferably from about 28,573bp to 28,760bp of the viral genome of SARS CoV-2, wherein the set of LAMP primers comprises a F3 primer comprising a nucleic acid which binds from about 28,550bp to 28,595bp, preferably from about 28,560bp to 28,594bp and most preferably from about 28,573bp to 28,593bp, such as, for example SEQ ID No: 32; a B3 primer comprising a nucleic acid which binds from about 28,744bp to 28,800bp, preferably from about 28,744bp to 28,780bp and most preferably from about 28,744bp to 28,760bp, such as, for example SEQ ID NO: 73; an FIP primer comprising a nucleic acid which binds from about 28,596bp to 28,666bp, preferably from about 28,596bp to 28,660bp and most preferably from about 28,596bp to 28,659bp, such as, for example SEQ ID NO: 36; and a BIP primer comprising a nucleic acid which binds from about 28,667bp to 28,743bp, preferably from about 28,670bp to 28,743bp and most preferably from about 28,674bp to 28,743bp, such as, for example SEQ ID NO: 72; and optionally comprising a Loop F primer comprising a nucleic acid which binds from about 28,637bp to 28,618bp, preferably from about 28,637bp to 28,620bp and most preferably from about 28,637bp to 28,621bp, such as, for example SEQ ID NO: 37, and a Loop B primer comprising a nucleic acid which binds from about 28,694bp to 28,722bp, preferably from about 28,700bp to 28,720bp and most preferably from about 28,705bp to 28,719bp, such as, for example SEQ ID NO: 70; of the viral genome of SARS CoV-2.

In a further preferred embodiment, a set of LAMP primers specifically amplifies a further SARS CoV-2 nucleic acid from about 28,450bp to 28,900bp, preferably from about 28,500bp to 28,800bp, or most preferably from about 28,515bp to 28,749bp of the viral genome of SARS CoV-2, wherein the set of LAMP primers comprises a F3 primer comprising a nucleic acid which binds from about 28,450bp to 28,544bp, preferably from about 28,500bp to 28,539bp and most preferably from about 28,515bp to 28,534bp, such as, for example SEQ ID No: 1; a B3 primer comprising a nucleic acid which binds from about 28,730bp to 28,850bp, preferably from about 28,732bp to 28,800bp and most preferably from about 28,735bp to 28,749bp, such as, for example SEQ ID NO: 68; an FIP primer comprising a nucleic acid which binds from about 28,544bp to 28,640bp, preferably from about 28,550b to 28,635bp and most preferably from about 28,554bp to 28,630bp, such as, for example SEQ ID NO: 4, or 5; and a BIP primer comprising a nucleic acid which binds from about 28,641bp to 28,729bp preferably from about 28,646bp to 28,727bp and most preferably from about 28,651bp to 28,726bp, such as, for example SEQ ID NO: 67 and optionally comprising a Loop F primer comprising a nucleic acid which binds from about 28,612bp to 28,570bp, preferably from about 28,605bp to 28,580bp and most preferably from about 28,597bp to 28,583bp, such as, for example SEQ ID NO: 12, or 13, and a Loop B primer comprising a nucleic acid which binds from about 28,671bp to 28,706bp, preferably from about 28,674bp to 28,700bp and most preferably from about 28,676bp to 28,692bp, such as, for example SEQ ID NO: 65; of the viral genome of SARS CoV-2.

In other embodiments, the means and methods include contacting a sample (such as a sample including or suspected to include SARS CoV-2 nucleic acids) with at least one set of LAMP primers specific for a SARS CoV-2 N-protein encoding nucleic acid (for example, a set of primers including the sequences of SEQ ID NOs: 1-18, or 31-56, or 64-73, 94, or 95) under conditions sufficient for amplification of the SARS CoV2 N gene nucleic acid, producing an amplification product. In some examples, the LAMP primers amplify a SARS CoV-2 N gene nucleic acid having at least 80% sequence identify (such as at least 85%, 90%, 95%, 98%, or more sequence identity) to nucleotides 28270-29530 of the SARS CoV-2 reference sequence, or a portion thereof. In some examples, the methods further include reverse transcription of SARS CoV-2 RNA in the sample, for example by contacting the sample with a reverse transcriptase. Contacting the sample with reverse transcriptase may be prior to contacting the sample with the one or more sets of LAMP primers, or may be simultaneous with contacting the sample with the one or more sets of LAMP primers (for example in the same reaction mix with the LAMP primers). The amplification product is detected by any suitable method, such as lateral flow dipsticks or detection of turbidity, fluorescence, or by gel electrophoresis.

In certain preferred embodiments of the invention the at least one primer in the set of RT-LAMP primers comprises a detectable label. A detectable label is preferably as one defined herein elsewhere. However, certain preferred labels are selected from any one of the following categories of labels: fluorophores, isotopes (e.g. 32P, 33P,35S, 3H, 14C, 1251, 1311), electron-dense reagents (e.g., gold, silver), nanoparticlcs, enzymes commonly used in an ELISA (e.g., horseradish peroxidase, beta-galactosidase, luciferase, alkaline phosphatase), chemiluminiscent compound, colorimetric labels (e.g., colloidal gold), magnetic labels (e.g., DynabeadsTM), biotin, digoxigenin, haptens, proteins for which antisera or monoclonal antibodies are available, ligands, hormones, oligonucleotides capable of forming a complex with the corresponding oligonucleotide complement, or any combination thereof. Most preferably the label used are the detectable labels indicated in table 1.

In certain embodiments the at least one set of LAMP primers may further comprise at least one quencher oligonucleotide.

The sample and LAMP primer set(s) are contacted under conditions sufficient for amplification of a SARS CoV-2 nucleic acid (such as an SARS CoV-2 ORF1AB or N-Protein nucleic acid). The sample is contacted with the set(s) of LAMP primers at a concentration sufficient to support amplification of an SARS CoV-2 nucleic acid. In some examples, the amount of each primer is about 0.1 µM to about 5 µM (such as about 0.2 µM to about 2 µM, or about 0.5 µM to about 2 µM). Each primer can be included at a different concentration, and appropriate concentrations for each primer can be selected by one of skill in the art using routine methods.

In some examples, the LAMP or RT-LAMP reaction is carried out in a mixture including a suitable buffer (such as a phosphate buffer or Tris buffer). The buffer may also include additional components, such as salts (such as KCl or NaCl, magnesium salts (e.g., MgCl2 or MgSO₄), ammonium (e.g., (NH₄)2SO₄)), detergents (e.g., TRITON^{®}-X100), or other additives (such as betaine, guanidine hydrochloride or dimethylsulfoxide). One of ordinary skill in the art can select an appropriate buffer and any additives using routine methods. In one non-limiting example, the buffer includes 20 mM Tris- HCl, 10 mM (NH₄)2SO₄, 50 mM KCl, 8 mM MgSO₄, 0.1% TRITON^{®}-X100, and 0.8 M betaine. An exemplary commercially available reaction buffer is IX Isothermal Amplification Buffer (New England Biolabs, Ipswich, MA). The reaction mixture also includes nucleotides or nucleotide analogs. In some examples, an equimolar mixture of dATP, dCTP, dGTP, and dTTP (referred to as dNTPs) is included, for example about 0.5-2 mM dNTPs. For the enhancement of the assay of the invention, in particular the addition of guanidine hydrochloride is preferred (see Zhang Y et al - https://doi.org/10.1101/2020.06.03.132894).

A DNA polymerase with strand displacement activity is also included in the reaction mixture. Exemplary DNA polymerases include Bst DNA polymerase, Bst 2.0 DNA polymerase, Bst 2.0 WarmStart^{™} DNA polymerase (New England Biolabs, Ipswich, MA), Phi29 DNA polymerase, Bsu DNA polymerase, OmniAmp^{™} DNA polymerase (Lucigen, Middleton, MI), Taq DNA polymerase, VentR^{®} and Deep VentR^{®} DNA polymerases (New England Biolabs), 9°Nm^{™} DNA polymerase (New England Biolabs), Klenow fragment of DNA polymerase I, PhiPRDl DNA polymerase, phage M2 DNA polymerase, T₄ DNA polymerase, and T₅ DNA polymerase. In some examples, about 1 to 20 U (such as about 1 to 15 U, about 2 to 12 U, about 10 to 20 U, about 2 to 10 U, or about 5 to 10 U) of DNA polymerase is included in the reaction. In some examples, the polymerase has strand displacement activity and lacks 5 '-3' exonuclease activity. In one non-limiting example, the DNA polymerase is Bst 2.0 WarmStart^{™} DNA polymerase (New England Biolabs, Ipswich, MA), for example aboutl6 U Bst 2.0 WarmStart^{™} DNA polymerase per reaction.

In some embodiments, the target SARS CoV-2 nucleic acid is RNA, and a reverse transcriptase is additionally included in the LAMP assay (called an RT-LAMP assay). Exemplary WarmStart^{®} RTx Reverse Transcriptase (NEB, Ipswich, MA), SuperScript IV Reverse Transcriptase (ThermoFisher Scientific, Waltham, MA), reverse transcriptases include MMLV reverse transcriptase, AMV reverse transcriptase, and ThermoScript^{™} reverse transcriptase (Life Technologies, Grand Island, NY), Thermo-X^{™} reverse transcriptase (Life Technologies, Grand Island, NY). In some examples, about 0.1 to 50 U (such as about 0.2 to 40 U, about 0.5 to 20 U, about 1 to 10 U, or about 2 to 5 U) of RT is included in the reaction. In a particular non-limiting example, the reaction includes 2 U/reaction of AMV RT.

The reaction mixture, including sample, LAMP primers, buffers, nucleotides, DNA polymerase, optionally reverse transcriptase and a second polymerase such as the Q₅^{®} High-Fidelity DNA Polymerase, and any other components, is incubated for a period of time and at a temperature sufficient for production of an amplification product. In some examples, the reaction conditions include incubating the reaction mixture at about 37°C to about 70°C (such as about 40°C-70°C, about 50°C-65°C, or about 45°C-60°C), for example about 40°C, about 45°C, about 50°C, about 55°C, about 60°C, about 65°C, or about 70°C. The reaction mixture is incubated for at least about 5 minutes (such as about 10, about 15, about 20, about 30, about 40, about 50, about 60, about 70, about 80 about 90, about 100, about 110, about 120 minutes or more), for example about 10-120 minutes, about 15-90 minutes, about 20-70 minutes, about 45-75 minutes, or about 30-60 minutes. In one non-limiting example, the reaction conditions include incubating the reaction mixture at about 60°C for about 60 minutes. The reaction can optionally be terminated by incubation for a short time at a higher temperature, for example about 2-10 minutes at about 80°C. In some examples, but not limited to, the assay is performed using a chemical heater device to maintain the sample at the reaction temperature for a period of time (see, e.g., Singleton et al., Proc. SPIE 8615:86250R, 2013; Curtis et al, PLoS ONE 7:e31432, 2012).

Following incubation of the reaction mixture, the amplification product is detected by any suitable method. The detection methods may be quantitative, semi-quantitative, or qualitative. Accumulation of an amplification product (for example, compared to a negative control, such as a reagent only control) indicates presence of SARS CoV-2 nucleic acids in the sample. In some examples, accumulation of an amplification product is detected by lateral flow immunochromatographic assays (for example, using dipsticks), by measuring the turbidity of the reaction mixture (for example, visually or with a turbidometer). In other examples, amplification product is detected using gel electrophoresis, for example by detecting presence or amount of amplification product with agarose gel electrophoresis. In some examples, amplification product is detected using a colorimetric assay, such as with a pH sensitive dye (for example phenol red) or an intercalating dye (for example, propidium iodide, SYBR green or Picogreen) in cycler-based realt-time analysis or a chromogenic reagent (see, e.g., Goto et al., BioTechniques 46: 167-172, 2009).

In further examples, amplification product is detected by a fluorescent indicator dye such as calcein or hydroxynaphthol blue (HNB)(see, e.g., Tomita et al., Nat. Protoc. 3:877-882, 2008). In other examples, amplification products are detected using a detectable label incorporated in one or more of the LAMP primers (discussed below). The detectable label may be optically detectable, for example, by eye or using a spectrophotometer or fluorimeter. In some examples, the detectable label is a fluorophore, such as those described above. In some examples, the label is detected in real-time, for example using a fluorescence scanner (such as ESEQuant, Qiagen). One of skill in the art can select one or more detectable labels for use in the methods disclosed herein.

Further, in a third aspect, the invention pertains to a method of diagnosing a corona virus infection in a subject, the method comprising detecting the presence of a corona virus nucleic acid in a biological sample previously obtained from the subject with a method of the first aspect, wherein the presence of the corona virus nucleic acid in the biological sample indicates a corona virus infection in the subject.

The methods of the herein disclosed invention are preferably ex-vivo, or in-vitro methods.

The methods of the invention are in preferred embodiments performed as a lateral flow test, preferably a lateral flow dipstick test.

### Primer and Kits

In **a second aspect,** the invention pertains to an isolated nucleic acid primer, comprising a nucleic acid sequence of any one of SEQ ID NOs: 1 to 73, 94, or 95or comprising a nucleic acid sequence having at least 80%, preferably 90%, sequence identity to any one of SEQ ID NO: 1 to 73 94, or 95, or comprising a sequence that hybridizes under stringent conditions to a target nucleic acid comprising a sequence which is complementary to any of SEQ ID NO: 1 to 73, 94, or 95.

In some embodiments, the disclosed primers are between 10 and 60 nucleotides in length, such as 10, 11, 12, 13, 14, 15, 16, 17, 18, 19, 20, 21, 22, 23, 24, 25, 26, 27, 28 29, 30, 31, 32, 32, 34, 35, 36, 37, 38, 39, 40, 41, 42, 43, 44, 45, 46, 47, 48, 49, 50, 51, 52, 53, 54, 55, 56, 57, 58, 59, or 60 nucleotides in length and are capable of hybridizing to, and in some examples, amplifying the disclosed nucleic acid molecules. In some examples, the primers and/or probes are at least 10, 15, 20, 25, 30, 35, 40, 45, 50, 55, or 60 nucleotides in length. In other examples, the primers and/or probes may be no more than 10, 15, 20, 25, 30, 35, 40, 45, 50, 55, or 60 nucleotides in length.

In some examples, the disclosed primers include LAMP primers for amplification of SARS CoV2 ORF1AB nucleic acids, including primers including a nucleic acid sequence with at least 90% sequence identity (for example, at least 91%, 92%, 93%, 94%, 95%, 96%, 97%, 98%, 99%, or 100% identity) to SEQ ID NO: 19, or 57 (F3), SEQ ID NOs: 26 (B3), SEQ ID NOs: 22 23, or 59 (FIP), SEQ ID NOs: 27, or 62 (BIP), SEQ ID NOs: 28 or 29 (LF), and/or SEQ ID NOs: 30 (LB), or the reverse complement of any thereof.

In some examples, the disclosed primers include LAMP primers for amplification of SARS CoV-2 N-gene nucleic acids, including primers including a nucleic acid sequence with at least 90% sequence identity (for example, at least 91%, 92%, 93%, 94%, 95%, 96%, 97%, 98%, 99%, or 100% identity) to SEQ ID NO: 1, 31, 32, 33, 40, or 47 (F3), SEQ ID NOs: 9, 38, 39, 56, 68 or 73 (B3), SEQ ID NOs: 4, 5, 6, 36, 44, 45, 51, or 95 (FIP), SEQ ID NOs: 10, 11, 17, 55, 67, or 72 (BIP), SEQ ID NOs: 12, 13, 37, 46, 49, or 94 (LF), and/or SEQ ID NOs: 14, 18, or 53, 65, or 70 (LB), or the reverse complement of any thereof.

In some examples, at least one of the primers includes a detectable label, such as a fluorophore. In particular examples, detectable labels as indicated in table 2 below.

Although exemplary primer sequences are provided herein, the primer sequences can be varied slightly by moving the primer a few nucleotides upstream or downstream from the nucleotide positions that they hybridize to on the target nucleic molecule acid, provided that the probe and/or primer is still specific for the target nucleic acid sequence. For example, variations of the primers disclosed as SEQ ID NOs: 1-73, 94, or 95 can be made by "sliding" the probes or primers a few nucleotides 5' or 3' from their positions, and such variations will still be specific for the respective target nucleic acid sequence.

Also provided by the present disclosure are primers that include variations to the nucleotide sequences shown in any of SEQ ID NOs: 1-73, as long as such variations permit detection of the target nucleic acid molecule. For example, a primer can have at least 90% sequence identity such as at least 91%, 92%, 93%, 94%, 95%, 96%, 97%, 98%, or 99% identity to a nucleic acid including the sequence shown in any of SEQ ID NOs: 1-73, 94, or 95. In such examples, the number of nucleotides does not change, but the nucleic acid sequence shown in any of SEQ ID NOs: 1-73, 94, or 95 can vary at a few nucleotides, such as changes at 1, 2, 3, 4, 5, or 6 nucleotides.

The present application also provides primers that are slightly longer or shorter than the nucleotide sequences shown in any of SEQ ID NOs: 1-73, 94, or 95, as long as such deletions or additions permit amplification and/or detection of the desired target nucleic acid molecule. For example, a primer can include a few nucleotide deletions or additions at the 5'- or 3 '-end of the primers shown in any of SEQ ID NOs: 1-73, 94, or 95, such as addition or deletion of 1, 2, 3, 4, 5, or 6 nucleotides from the 5'- or 3 '-end, or combinations thereof (such as a deletion from one end and an addition to the other end). In such examples, the number of nucleotides changes.

Also provided are primers that are degenerate at one or more positions (such as 1, 2, 3, 4, 5, or more positions), for example, a primer that includes a mixture of nucleotides (such as 2, 3, or 4 nucleotides) at a specified position in the primer. In other examples, the primers disclosed herein include one or more synthetic bases or alternative bases (such as inosine). In other examples, the primers disclosed herein include one or more modified nucleotides or nucleic acid analogues, such as one or more locked nucleic acids (see, e.g., U.S. Pat. No. 6,794,499) or one or more superbases (Nanogen, Inc., Bothell, WA). In other examples, the primers disclosed herein include a minor groove binder conjugated to the 5' or 3' end of the oligonucleotide (see, e.g., U.S. Pat. No. 6,486,308).

The nucleic acid primers disclosed herein can be supplied in the form of a kit or a kit for use in the detection or amplification of one or more SARS CoV-2 nucleic acids. Hence, in a **fourth aspect,** the invention pertains to a diagnostic kit for use in the detection of a corona virus nucleic acid, comprising one or more of the isolated nucleic acid primers of the invention.

In **a fifth aspect,** the invention pertains to a use of the isolated nucleic acid primer of the third aspect or the diagnostic kit of the second aspect in a method of detecting corona virus nucleic acids, preferably according to the first or third aspect.

In such a kit, an appropriate amount of one or more of the nucleic acid primers (such as one or more of SEQ ID NOs: 1-73, 94, or 95) are provided in one or more containers or in one or more individual wells or channels of a multiwell plate or card or a microfluidic device. A nucleic acid primer(s) may be provided suspended in an aqueous solution or as a freeze-dried or lyophilized powder, for instance. The container(s) in which the nucleic acid(s) are supplied can be any conventional container that is capable of holding the supplied form, for instance, microfuge tubes, multi-well plates, ampoules, or bottles. The kits can include either labeled or unlabeled nucleic acid primers (for example, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30 or more primers) for use in amplification and/or detection of SARS CoV-2 nucleic acids. One or more positive and/or negative control and internal control primers and/or nucleic acids also may be supplied in the kit. Exemplary negative controls include non- SARS-CoV-2 nucleic acids (such as HCoV-OC43 or HCoV-229E nucleic acids) or non- viral nucleic acids (such as human nucleic acids). Exemplary positive controls include primers and nucleic acids for amplification of human target nucleic acids (such as human β-actin or RNaseP) or primers and nucleic acids for other SARS-CoV-2 target nucleic acids. Exemplary internal controls include primers and nucleic acids for amplification of non-human infecting viral nucleic acids (such as plant pathogen potato virus X, PVX). One of ordinary skill in the art can select suitable positive and negative controls for the assays disclosed herein.

In some examples, one or more primers (such as one or more sets of primers), are provided in pre-measured single use amounts in individual, typically disposable, tubes, wells, microfluidic devices, or equivalent containers. In this example, the sample to be tested for the presence of the target nucleic acids can be added to the individual tube(s) or well(s) and amplification and/or detection can be carried out directly. The kit may also include additional reagents for the detection and/or amplification of SARS CoV-2 nucleic acids, such as buffer(s), nucleotides (such as dNTPs), enzymes (such as DNA polymerase(s) and/or reverse transcriptase), or other suitable reagents. The additional reagents may be in separate container(s) from the one or more primers or may be included in the same container as the primer(s).

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES AND SEQUENCES

The figures show:
**Figure 1**: shows a schematic of a standard RT-LAMP probe set (left side) in comparison to a classical RT-PCR primer set.
**Figure 2**: shows a comparison of corona viral genome architecture according to Wu et al 2020 Nature Vol 579).
**Figure 3**: shows the mapping of annotated LAMP primers of the invention in the ORF1a/b genomic location of SARS CoV-2.

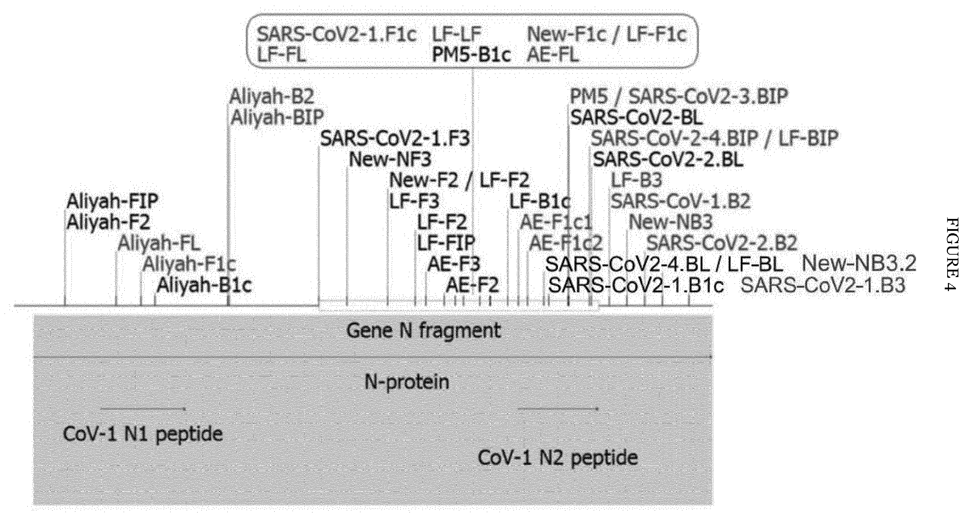

**Figure 4****:** shows the mapping of annotated LAMP primers of the invention in the N gene genomic location of SARS CoV-2.
**Figure 5****:** shows the O1a/b LAMP primer binding sites of the annotated primer in the ORF1a/b genomic region of SARS CoV-2.
**Figure 6**: shows an overview of the preferred LAMP primer sets CLPS1 and CLPS2, as annotated, of the present invention in the SARS-CoV-2 N gene.
**Figure 7****:** shows the primer binding sites of the CLPS1 primer set in the N gene genomic region of SARS CoV-2.
**Figure 8****:** shows the primer binding sites of the CLPS2 primer set in the N gene genomic region of SARS CoV-2.
**Figure 9**: shows a time-to-result comparison between SARS-CoV-2 LAMP primer sets of the present invention and shows already achieved optimizations between the LAMP Primer Sets of the present invention.
**Figure 10**: shows a time-to-result comparison between published E and N primer sets of NEB (https://doi.org/10.1101/2020.06.03.132894) and the inventive SARS-CoV-2 LAMP Primer Sets of the present invention.
**Figure 11**: shows Ct values of SARS CoV-2 primer test with the SARS-CoV2 standard sample RV340059 using six different primer dilutions ranging from undiluted to 1:1,000.
**Figure 12**: shows Tm values of SARS CoV-2 primer test with the SARS-CoV2 standard sample RV340059 using six different primer dilutions ranging from undiluted to 1:1,000.
**Figure 13**: shows Ct values of SARS CoV-2 primer mix comparison using six different primers in serial dilution of clinical samples in GPS. Dilutions ranging from 1:4 to 1:512.
**Figure 14**: shows Tm values of SARS CoV-2 primer mix comparison using six different primers in serial dilution of clinical samples in GPS. Dilutions ranging from 1:4 to 1:512.
**Figure 15**: shows Ct values of SARS CoV-2 reference samples 1 and 2 in serial dilutions from undiluted to 1:128 by using the three selected primer sets CLPS1, CLPS3 and CLPS2 each in a duplex reaction with the control (ACTB).
**Figure 16**: shows Tm values of SARS CoV-2 reference samples 1 and 2 in serial dilutions from undiluted to 1:128 by using the three selected primer sets CLPS1, CLPS3 and CLPS2 each in a duplex reaction with the control (ACTB). Filled bars are positive for the control (ACTB) and SARS CoV-2, whereby the green bars were additionally verified via a lateral flow dipstick. Striped bars are positive for SARS CoV-2, but indicate no amplification of actin. Non-filled bars are negative for SARS CoV-2.
**Figure 17**: shows Ct value of SARS CoV-2 triplex for determination of primer concentration with CLPS1, CLPS2, and the control (ACTB), and reference sample 1 in serial dilution of 1000 copies/µL to 31.25 copies/µL.
**Figure 18**: shows Ct value of SARS CoV-2 triplex-duplex comparison, reference sample 1 in serial dilution of 1000 copies/µL to 31.25 copies/µL, negative control: 2×Stabilizing buffer.
**Figure 19**: shows Ct value of SARS CoV-2, ACTB concentration in Triplex, RV340080 and RV3400077 dilutions, CLPS1 and CLPS2.
**Figure 20**: shows melting curve analysis of SARS CoV-2 LAMP in accordance of the present invention.

The sequences show:

**Table 2: RT-LAMP Oligonucleotides of the Invention**

| **Designation:** | **Sequence:** | **Target SARS-CoV2 Genomic Coordinates:** | **SEQ ID NO:** |
|---|---|---|---|
| SARS-CoV2-1.F3 | ATGACCAAATTGGCTACT AC | 28515-28534 | 1 |
| SARS-CoV2-1.F2 | TTCGTGGTGGTGACGG | 28554-28569 | 2 |
| SARS-CoV2-1.F1c | AGCTTCTGGCCCAGTTCC | 28630-28613 | 3 |
| SARS-CoV2-1.FIP | AGCTTCTGGCCCAGTTCC TTCGTGGTGGTGACGG | 28630-28613; 28554-28569 | 4 |
| Biotin- SARS-CoV2-1.FIP | AGCTTCTGGCCCAGTTCC TTCGTGGTGGTGACGG | Biotin-28630-28613; 28554-28569 | 5 |
| JOE- SARS-CoV2-1.FIP | JOE- AGCTTCTGGCCCAGTTCC TTCGTGGTGGTGACGG | JOE-28630-28613; 28554-28569 | 6 |
| SARS-CoV2-1.B1c | ATTGGCACCCGCAATCC | 28709-28725 | 7 |
| SARS-CoV2-1.B2 | GAGGAAGTTGTAGCAC | 28760-28745 | 8 |
| SARS-CoV2-1.B3 | CGAGAAGAGGCTTGACT | 28827-28811 | 9 |
| SARS-COV2-1.BIP | ATTGGCACCCGCAATCCG AGGAAGTTGTAGCAC | 28709-28725;28760-28745 | 10 |
| DIG-SARS-COV2-1.BIP | ATTGGCACCCGCAATCCG AGGAAGTTGTAGCAC | DIG-28709-28725; 28760-28745 | 11 |
| SARS-CoV2-1.FL | CCATCTTGGACTGAG | 28597-28583 | 12 |
| FAM- SARS-CoV2-1.FL | FAM- CCATCTTGGACTGAG | FAM -28597-28583 | 13 |
| SARS-CoV2-1.BL | TGCTAACAATGCTGC | 28726-28740 | 14 |
| SARS-CoV2-2.B1c | ATTGGCACCCGCAATCC | 28709-28725 | 15 |
| SARS-CoV2-2.B2 | CGTAGAAGCCTTTTGGCA AT | 28790-28771 | 16 |
| SARS-CoV2-2.BIP | ATTGGCACCCGCAATCCC GTAGAAGCCTTTTGGCAA T | 28709-28725,28790-28771 | 17 |
| SARS-CoV2-2.BL | GTGCTACAACTTCCTC | 28745-28760 | 18 |
| 1.ORF1ab-F3 | ATGGCACTTGTGGCTTAG TAGA | 408-429 | 19 |
| 1.ORF1ab-F2 | CTTGAACAGCCCTATGTG | 455-472 | 20 |
| 1.ORF1ab-F1c | CTACGACCGTACTGAATG CC | 564-545 | 21 |
| 1.ORF1ab-FIP | CTACGACCGTACTGAATG CCCTTGAACAGCCCTATG TG | 564-545; 455-472 | 22 |
| DIG-1.ORF1ab-FIP | DIG- CTACGACCGTACTGAATG CCCTTGAACAGCCCTATG TG | DIG-564-545; 455-472 | 23 |
| 1.ORF1ab-B1c | CCTCATGTGGGCGAAA | 589-605 | 24 |
| 1.ORF1ab-B2 | CCTAAGTCAAATGACTTT AGATCG | 701-679 | 25 |
| 1.ORF1ab-B3 | AGCTCACGCATGAGTTCA CGG | 795-775 | 26 |
| 1.ORF1ab-BIP | CCTCATGTGGGCGAAACC TAAGTCAAATGACTTTAG ATCG | 589-605; 701-679 | 27 |
| 1.ORF1ab-FL | TTCTGCTACCAGCTCAAC | 538-521 | 28 |
| FAM-1.ORF1ab-FL | TTCTGCTACCAGCTCAAC | FAM-538-521 | 29 |
| 1.ORF1ab-BL | TACCGCAAGGTTCTTCTTC G | 617-636 | 30 |
| New-N.F3 | GAGCTACCAGACGAA | 28539-28553 | 31 |
| LF-F3 | AATGAAAGATCTCAGTCC AAG | 28573-28593 | 32 |
| LF-F3.2 | AGATCTCAGTCCAAG | 28579-28593 | 33 |
| LF-F2 | GGTATTTCTACTACCTAG GAAC | 28596-28617 | 34 |
| New-Fic_compl. | CCGTCTTTGTTAGCACCAT AGG | 28659-28638 | 35 |
| Biotin-LF-FIP | Biotin- CCGTCTTTGTTAGCACCAT AGGGGTATTTCTACTACC TAGGAAC | Biotin-28659-28638; 28596-28617 | 36 |
| FAM-LF-FL | FAM- GAAGTCCAGCTTCTGGC | FAM -28637-28621 | 37 |
| New-N.B3 | GCAATGTTGTTCCTT | 28775-28761 | 38 |
| New-N.B3.2 | CTCTGCTCCCTTCTG | 28805-28791 | 39 |
| AE-F3 | ACTACCTAGGAACTGG | 28605-28620 | 40 |
| AE-F2 | GCCAGAAGCTGGACTTCC CTAT | 28621-28642 | 41 |
| AE-F1c | CCCTCAGTTGCAACCCAT ATG | 28683-28663 | 42 |
| AE-F1c2 | TCAAGGCTCCCTCAGTTG C | 28691-28673 | 43 |
| Biotin-AE-FIPi | Biotin- CCCTCAGTTGCAACCCAT ATGGCCAGAAGCTGGACT TCCCTAT | Biotin-28683-28663; 28621-28642 | 44 |
| Biotin-AE-FIP2 | Biotin- TCAAGGCTCCCTCAGTTG CGCCAGAAGCTGGACTTC CCTAT | Biotin-28691-28673; 28621-28642 | 45 |
| FAM-AE-FL | FAM- GCCGTCTTTGTTAGC | FAM -28660-28646 | 46 |
| Aliyah-F3 | GGACCCCAAAATCAG | 28286-28300 | 47 |
| Aliyah-F2 | CGAAATGCACCCCGCATT AC | 28301-28320 | 48 |
| FAM-Aliyah-FL | FAM- GTTGAATCTGAGGGTCC | FAM-28344-28328 | 49 |
| Aliyah-Fic | TCTCCATTCTGGTTACTGC C | 28365-28346 | 50 |
| Biotin-Aliyah-FIP | Biotin- TCTCCATTCTGGTTACTGC CCGAAATGCACCCCGCAT TAC | Biotin-28365-28346; 28301-28320 | 51 |
| Aliyah-B1c | CGCGATCAAAACAACGTC GG | 28377-28396 | 52 |
| Aliyah-BL | CCCCAAGGTTTACCC | 28397-28411 | 53 |
| Aliyah-B2 | AGCGGTGAACCAAGACGC AG | 28438-28419 | 54 |
| Aliyah-BIP | CGCGATCAAAACAACGTC GGAGCGGTGAACCAAGAC GCAG | 28377-28396; 28438-28419 | 55 |
| Aliyah-B3 | CTTGCCATGTTGAGTG | 28457-28441 | 56 |
| O1a/b-NewF3 | ACGTTCGGATGCTCG | 481-495 | 57 |
| O1a/b-NewF2 | ACTGCACCTCATGGTCAT GT | 497-516 | 58 |
| DIG-O1a/b-NewFIP | DIG- CTACGACCGTACTGAATG CCACTGCACCTCATGGTC ATGT | DIG-564-545; 497-516 | 59 |
| O1a/b-NewB1c | GTGGGCGAAATACCAGTG GC | 596-615 | 60 |
| O1a/b-NewB2_1408 | CCAGCTCCTTTATTACCGT T | 660-640 | 61 |
| O1ab-NewBIP_1408 | GTGGGCGAAATACCAGTG GCCCAGCTCCTTTATTACC GTT | 596-615; 660-640 | 62 |
| O1ab-NewB3_1408 | GCGCCGTAACTATGG | 678-664 | 63 |
| SARS-CoV2-3.B1c | CAAAGACGGCATCATATG GG | 28651-28670 | 64 |
| SARS-CoV2-3.BL | ACTGAGGGAGCCTTGA | 28676-28691 | 65 |
| SARS-CoV2-3.B2 | GGATTGCGGGTGCCAATG TG | 28725-28706 | 66 |
| SARS-CoV2-3.BIP | CAAAGACGGCATCATATG GGGGATTGCGGGTGCCAA TGTG | 28651-28670; 28725-28706 | 67 |
| SARS-CoV2-3.B3 | AGCACGATTGCAGCAT | 28749-28734 | 68 |
| SARS-CoV-2-4.B1c | CAACTGAGGGAGCCTTGA AT | 28674-28693 | 69 |
| SARS-CoV2-4.BL / LF-BL | TCACATTGGCACCCG | 28705-28719 | 70 |
| SARS-CoV-2-4.BIP / LF-B2 | TGCAGCATTGTTAGCAGG | 28743-28724 | 71 |
| SARS-CoV-2-4.BIP / LF-BIP | CAACTGAGGGAGCCTTGA ATTGCAGCATTGTTAGCA GG | 28674-28693; 28743-28724 | 72 |
| LF-B3 | GAGGAAGTTGTAGCAC | 28760-28744 | 73 |
| ACTB- F3 | AGTACCCCATCGAGCACG | - | 74 |
| DIG-ACTB-FIP | DIG- GAGCCACACGCAGCTCAT TGTATCACCAACTGGGAC GACA | - | 75 |
| FAM-ACTB-FL | TGTGGTGCCAGATTTTCT CCA | - | 76 |
| ACTB-BL | CGAGAAGATGACCCAGAT CATGT | - | 77 |
| ACTB-BIP | CTGAACCCCAAGGCCAAC CGGCTGGGGTGTTGAAGG TC | - | 78 |
| ACTB-B3 | AGCCTGGATAGCAACGTA CA | - | 79 |
| Aliyah-FL | GTTGAATCTGAGGGTCC | 28344-28328 | 94 |
| Aliyah-FIP | TCTCCATTCTGGTTACTGC CCGAAATGCACCCCGCAT TAC | 28365-28346; 28301-28320 | 95 |

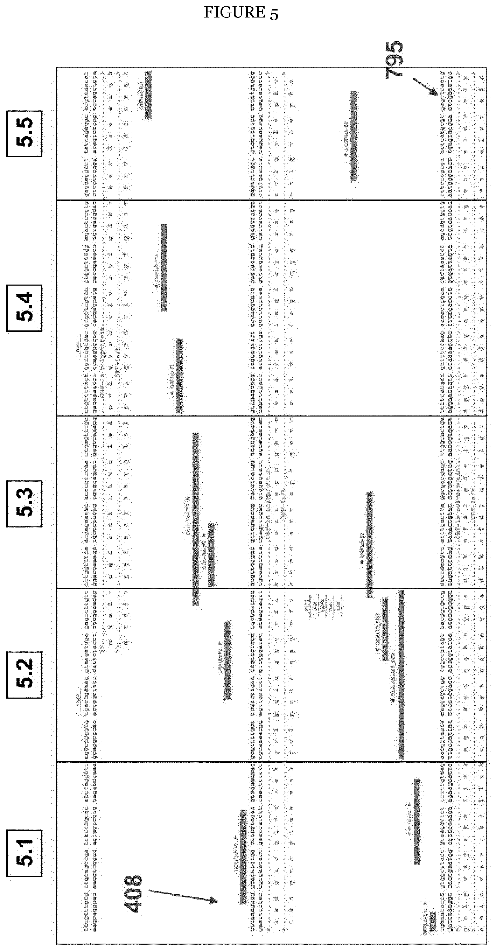

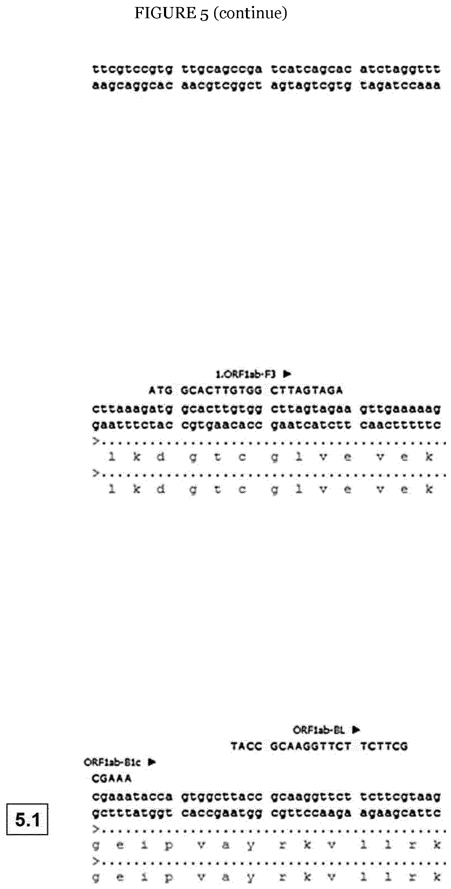

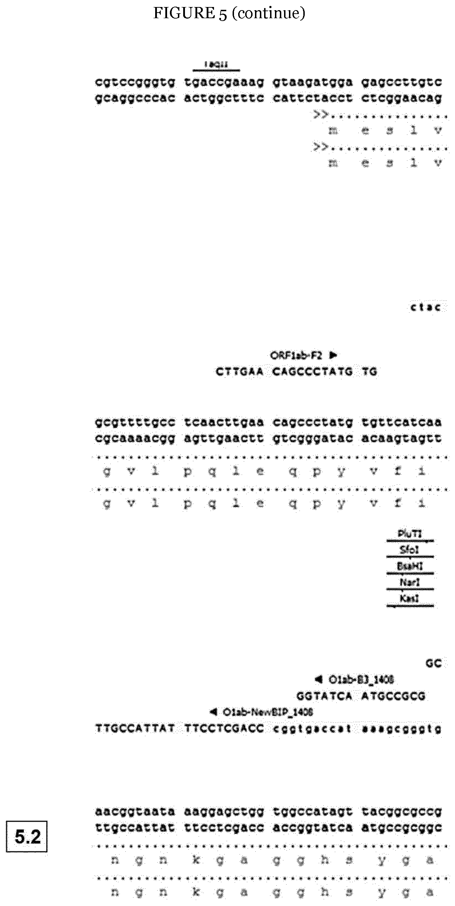

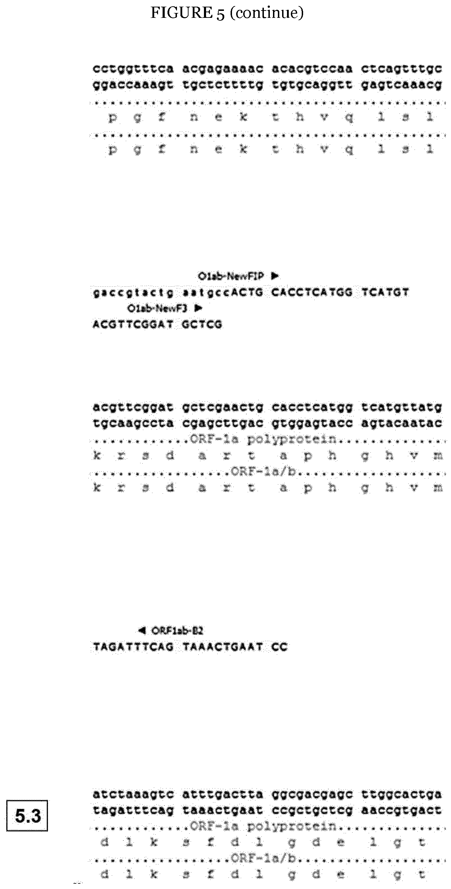

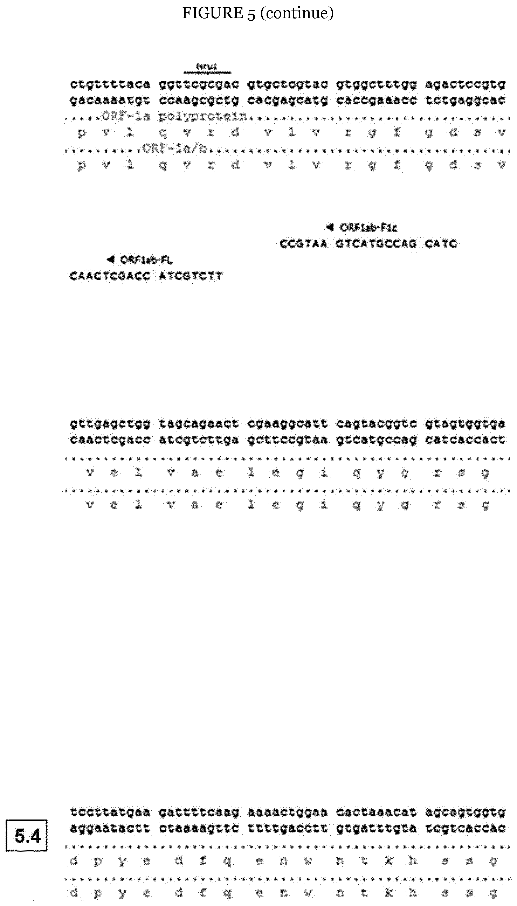

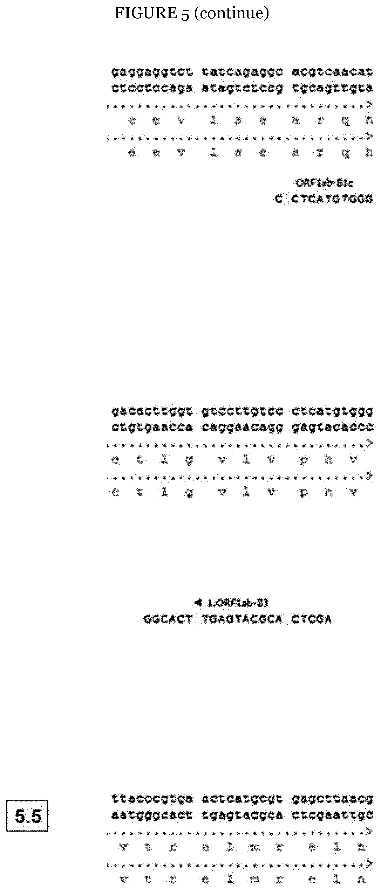

SEQ ID NO: 80 in Figure 5 is a fragment of the ORF1ab genomic region of SARS-CoV2 (SEQ ID NO: 80):

TTCGTCCGTGTTGCAGCCGATCATCAGCACATCTAGGTTTCGTCCGGGTGTGACCGAAAGGTA
AGATGGAGAGCCTTGTCCCTGGTTTCAACGAGAAAACACACGTCCAACTCAGTTTGCCTGTTTT
ACAGGTTCGCGACGTGCTCGTACGTGGCTTTGGAGACTCCGTGGAGGAGGTCTTATCAGAGGC
ACGTCAACATCTTAAAGATGGCACTTGTGGCTTAGTAGAAGTTGAAAAAGGCGTTTTGCCTCA
ACTTGAACAGCCCTATGTGTTCATCAAACGTTCGGATGCTCGAACTGCACCTCATGGTCATGTT
ATGGTTGAGCTGGTAGCAGAACTCGAAGGCATTCAGTACGGTCGTAGTGGTGAGACACTTGGT
GTCCTTGTCCCTCATGTGGGCGAAATACCAGTGGCTTACCGCAAGGTTCTTCTTCGTAAGAACG
GTAATAAAGGAGCTGGTGGCCATAGTTACGGCGCCGATCTAAAGTCATTTGACTTAGGCGACG
AGCTTGGCACTGATCCTTATGAAGATTTTCAAGAAAACTGGAACACTAAACATAGCAGTGGTG
TTACCCGTGAACTCATGCGTGAGCTTAACG

SEQ ID NO: 81 shows the complementary sequence of the fragment of the ORF1ab genomic region of SARS-CoV2 (SEQ ID NO: 81):

AAGCAGGCACAACGTCGGCTAGTAGTCGTGTAGATCCAAAGCAGGCCCACACTGGCTTTCCAT
TCTACCTCTCGGAACAGGGACCAAAGTTGCTCTTTGTGTGCAGGTTGAGTCAAACGGACAAA
ATGTCCAAGCGCTGCACGAGCATGCACCGAAACCTCTGAGGCACCTCCTCCAGAATAGTCTCCG
TGCAGTTGTAGAATTTCTACCGTGAACACCGAATCATCTTCAACTTTTTCCGCAAAACGGAGTT
GAACTTGTCGGGATACACAAGTAGTTTGCAAGCCTACGAGCTTGACGTGGAGTACCAGTACAA
TACCAACTCGACCATCGTCTTGAGCTTCCGTAAGTCATGCCAGCATCACCACTCTGTGAACCAC
AGGAACAGGGAGTACACCCGCTTTATGGTCACCGAATGGCGTTCCAAGAAGAAGCATTCTTGC
CATTATTTCCTCGACCACCGGTATCAATGCCGCGGCTAGATTTCAGTAAACTGAATCCGCTGCT
CGAACCGTGACTAGGAATACTTCTAAAAGTTCTTTGACCTTGTGATTTGTATCGTCACCACAA
TGGGCACTTGAGTACGCACTCGAATTGC

SEQ ID NO: 82 in Figure 5 is a fragment of the ORF1a polyprotein of SARS-CoV2 (SEQ ID NO: 82):

MESLVPGFNEKTHVQLSLPVLQVRDVLVRGFGDSVEEVLSEARQHLKDGTCGLVEVEKGVLPQ
LEQPYVFIKRSDARTAPHGHVMVELVAELEGIQYGRSGETLGVLVPHVGEIPVAYRKVLLRKNG
NKGAGGHSYGADLKSFDLGDELGTDPYEDFQENWNTKHSSGVTRELMRELN

SEQ ID NO: 83 in Figure 5 is a fragment of the ORF1a/b polyprotein of SARS-CoV2 (SEQ ID NO: 83):

MESLVPGFNEKTHVQLSLPVLQVRDVLVRGFGDSVEEVLSEARQHLKDGTCGLVEVEKGVLPQ
LEQPYVFIKRSDARTAPHGHVMVELVAELEGIQYGRSGETLGVLVPHVGEIPVAYRKVLLRKNG
NKGAGGHSYGADLKSFDLGDELGTDPYEDFQENWNTKHSSGVTRELMRELN

SEQ ID NO: 84 in Figure 7 is a fragment of the N gene genomic region of SARS-CoV2 (SEQ ID NO: 84):

GACCCCAAAATCAGCGAAATGCACCCCGCATTACGTTTGGTGGACCCTCAGATTCAACTGGCA
GTAACCAGAATGGAGAACGCAGTGGGGCGCGATCAAAACAACGTCGGCCCCAAGGTTTACCCA
ATAATACTGCGTCTTGGTTCACCGCTCTCACTCAACATGGCAAGGAAGACCTTA

SEQ ID NO: 85 shows the complementary sequence of the N gene genomic region of SARS-CoV2 (SEQ ID NO: 85):

CTGGGGTTTAGTCGCTTTACGTGGGGCGTAATGCAAACCACCTGGGAGTCTAAGTTGACCGT
CATTGGTCTTACCTCTTGCGTCACCCCGCGCTAGTTTTGTTGCAGCCGGGGTTCCAAATGGGTT
ATTATGACGCAGAACCAAGTGGCGAGAGTGAGTTGTACCGTTCCTTCTGGAAT

SEQ ID NO: 86 in Figure 7 shows the following sequence that is a T7-promoter sequence (SEQ ID NO: 86): TAATACGACTCACTATAGGG

SEQ ID NO: 87 in Figure 7 is a T7-promoter sequence (SEQ ID NO: 87): ATTATGCTGAGTGATATCCC

SEQ ID NO: 88 in Figure 7 is a fragment of the N protein of SARS-CoV2 (SEQ ID NO: 88): GPQNQRNAPRITFGGPSDSTGSNQNGERSGARSKQRRPQGLPNNTASWFTALTQHGKEDL

SEQ ID NO: 89 in Figure 7 is a fragment of the N1 peptide of SARS-CoV-1 (SEQ ID NO: 89): PSDSTGSNQNGERSGARSKQRRPQ

SEQ ID NO: 90 in Figure 8 is a fragment of the N gene genomic region of SARS-CoV2 (SEQ ID NO: 90):

AATTCCCTCGAGGACAAGGCGTTCCAATTAACACCAATAGCAGTCCAGATGACCAAATTGGCTA
CTACCGAAGAGCTACCAGACGAATTCGTGGTGGTGACGGTAAAATGAAAGATCTCAGTCCAAG
ATGGTATTTCTACTACCTAGGAACTGGGCCAGAAGCTGGACTTCCCTATGGTGCTAACAAAGA
CGGCATCATATGGGTTGCAACTGAGGGAGCCTTGAATACACCAAAAGATCACATTGGCACCCG
CAATCCTGCTAACAATGCTGCAATCGTGCTACAACTTCCTCAAGGAACAACATTGCCAAAAGGC
TTCTACGCAGAAGGGAGCAGAGGCGGCAGTCAAGCCTCTTCTCGTTCCTCATCACGTAGTCGC
AACAGTTCAAGAAATTCAAC

SEQ ID NO: 91 in Figure 8 is a fragment of the N gene genomic region of SARS-CoV2 (SEQ ID NO: 91):

TTAAGGGAGCTCCTGTTCCGCAAGGTTAATTGTGGTTATCGTCAGGTCTACTGGTTTAACCGAT
GATGGCTTCTCGATGGTCTGCTTAAGCACCACCACTGCCATTTACTTTCTAGAGTCAGGTTCT
ACCATAAAGATGATGGATCCTTGACCCGGTCTTCGACCTGAAGGATACCACGATTGTTTCTGC
CGTAGTATACCCAACGTTGACTCCCTCGGAACTTATGTGGTTTTCTAGTGTAACCGTGGGCGTT
AGGACGATTGTTACGACGTTAGCACGATGTTGAAGGAGTTCCTTGTTGTAACGGTTTTCCGAA
GATGCGTCTTCCCTCGTCTCCGCCGTCAGTTCGGAGAAGAGCAAGGAGTAGTGCATCAGCGTT
GTCAAGTTCTTTAAGTTG

SEQ ID NO: 92 in Figure 8 is a fragment of the N protein of SARS-CoV2 (SEQ ID NO: 92):

KFPRGQGVPINTNSSPDDQIGYYRRATRRIRGGDGKMKDLSPRWYFYYLGTGPEAGLPYGANK
DGIIWVATEGALNTPKDHIGTRNPANNAAIVLQLPQGTTLPKGFYAEGSRGGSQASSRSSSRSR
NS

SEQ ID NO: 93 in Figure 8 is a fragment of the N2 peptide of SARS-CoV1 (SEQ ID NO: 93): EPIHQKITLAPAILLTMLQSCY

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Development of SARS CoV-2 LAMP Primers

In the relevant technical literature and by the leading manufacturers of RT-LAMP kits (NEB, Optigene^{®}, Lucigene^{®}) the free web-based program "Primer Explorer" (EIKEN Chemical Co., Ltd.) is recommended for the design of the probes. The user first enters well-preserved target gene sections into an online mask, which then algorithmically recommends 3-5 suggestions for LAMP primer sets consisting of F3, B3, FIP and BIP probes. The so-called loop primers, which are not generated in this application (Fig. 1**:** FL and BL oligos) are however essential. It has been proven that loop primers not only increase the specificity of the reaction, but also significantly (up to 30 %) reduce the reaction time (time-to-result / time-to-threshold, Ct/q-value). However, these FL / BL probes must be generated separately in a second step using the immature, end-user-unfriendly app (including multiple bugs / program errors).

However, the inventor's experiences as well as numerous publications clearly prove that freely available programs such as the "Primer Explorer" or comparable software applications (e.g. LAMP Designer) are very faulty. Fine details, which are an absolute prerequisite for a successful LAMP reaction, such as the efficient exclusion of mismatches, primer dimers and suboptimal hairpins, are not sufficiently taken into account by these programs.

After intensive testing and evaluation of various LAMP probes against different RNA viruses (as well as the coronavirus SARS-CoV-2) derived by means of the above-mentioned web-based program "Primer Explorer", it was found that they always caused an unacceptable abundance of false-positive and false-negative results. In some cases, the program even generated primers that had no specificity for the viral target sequence.

In the present research, the inventors therefore developed highly specific probes (see table 2) for the rapid and above all reliable identification of a SARS CoV-2 infection using RT-LAMP and confirmed their precision in clinical analysis laboratories. This has laid the foundation for the first time for the sustainable use of this method in clinical diagnostics.

The development of highly specific LAMP probes against SARS-CoV-2 was based on the following approach: first, highly conserved regions of the viral target sequence (up to 300 bp) were identified by our own extensive bioinformatic comparative analyses of all published genomes and these were each divided into eight separate base sections. These 15 to 25 long oligomeric regions have the best possible distances to each other [F2-F3: 0-60 nt, F1c-B1c: 0-100 nt, F2-B2: 120-160 nt; F1c-F2 (loops): 40-60 nt] and have a melting temperature (Tm) between 55°C and 68°C, with a maximum difference of 5°C. The melting temperature characterizes the point at which 50% of the oligonucleotides are bound to their target sequence and 50% are free in solution. Ideally these should be in the range of the RT-LAMP reaction temperature (63°-68°C).

In order to ensure robust hybridization and consequently error-free amplification of the target sequence, only high, negative ΔG° values with respect to the terminal 5' and especially 3' end of the detection oligonucleotides were considered (at least -4 kcal/mol, but better ≥ -5 kcal/mol). The primers also have a GC content between 40-60% and are not directed against repetitive sequence regions to avoid false amplifications. Regions that could potentially form secondary structures as well as intra-primer homologies (more than 3 bases complementary to each other within the oligonucleotide) or inter-primer homologies (sense and antisense primers have complementary segments) were also identified and excluded (or modified using state-of-the-art methods). For the final check of the specificity of the LAMP products using the lateral flow dipstick method, the oligonucleotides still have the following labels: FL probe: FAM marking at the 5' end; FIP probe: Biotin or digoxigenin label at the 5' end; BIP probe: JOE at the 5' end.

Table 3 below lists exemplary preferred primer sets and their names as used to designate the RT-LAMP primer sets in the following experiments of the present invention. However, said

Table 3 is not intended to be restrictive, as individual primers listed below from a named set can also be combined with other primers from other sets if they are suitable.

**Table 3: Overview of preferred primer sets CLPS1 to 7 including the ACTB control primer set according to the present invention with respective primer and their corresponding SEQ ID Nos..**

| Primer set | Primer Designation | SEQ ID NO |
|---|---|---|
| Control (ACTB) | ACTB- F3 | 74 |
| | DIG-ACTB-FIP | 75 |
| | FAM-ACTB-FL | 76 |
| | ACTB- BL | 77 |
| | ACTB- BIP | 78 |
| | ACTB- B3 | 79 |
| CLPS1 | Aliyah-F3 | 47 |
| | Aliyah-FIP | 51 |
| | Aliyah-FL | 49 |
| | Aliyah-BL | 53 |
| | Aliyah-BIP | 55 |
| | Aliyah-B3 | 56 |
| CLPS2 | SARS-CoV2-1.F3 | 1 |
| | Biotin-SARS-CoV2-i.FIP | 5 |
| | FAM-SARS-CoV2-1.FL | 13 |
| | SARS-CoV2-3.BL | 65 |
| | SARS-CoV2-3.BIP | 67 |
| | SARS-CoV2-3.B3 | 68 |
| CLPS3 | LF-F3 | 32 |
| | FAM-LF-FL | 37 |
| | Biotin-LF-FIP | 36 |
| | SARS-CoV2-1.BL/ LF-BL | 70 |
| | SARS-CoV2-1.BIP/ LF-BIP | 72 |
| | SARS-CoV2-4.B3/ LF-B3 | 73 |
| CLPS4 | AE-F3 | 40 |
| | Biotin-AE-FIP2 | 45 |
| | FAM-AE-FL | 46 |
| | SARS-CoV2-1.BL | 14 |
| | SARS-CoV2-1.BIP | 10 |
| | New-NB3 | 38 |
| CLPS5 | O1a/b-NewF3 | 57 |
| | O1a/b-NewFIP | 59 |
| | FAM-1. ORF1ab-FL | 29 |
| | 1. ORF1ab-BL | 30 |
| | O1a/b-NewBIP_1408 | 62 |
| | O1a/b-NewB3_1408 | 63 |
| CLPS6 | SARS-CoV2.1.-F3 | 1 |
| | Biotin-SARS-CoV2.1.-FIP | 5 |
| | FAM-SARS-CoV2.1.-FL | 13 |
| | SARS-CoV2.1.-BL | 14 |
| | SARS-CoV2.1.-BIP | 10 |
| | SARS-CoV2.1.-B3 | 9 |
| CLPS7 | 1.ORF1ab -F3 | 19 |
| | 1.ORF1ab -FIP | 22 |
| | 1.ORF1ab -FL | 28 |
| | 1.ORF1ab-BL | 30 |
| | 1.ORF1ab-BIP | 27 |
| | 1.ORF1ab-B3 | 26 |

The RT-LAMP experiments described herein were performed with the WarmStart^{®} LAMP Kit (DNA & RNA) purchased from New England Biolabs (NEB). All necessary reagents for performing the RT-LAMP were included in this commercially available kit. Further, the experiments described herein were performed according to known standard protocols for LAMP procedures.**Example 2:** RING Testing ("Ringversuch") of the Developed LAMP Primers

The validation of the developed LAMP primers was carried out on the basis of ring samples from the company Instand e.V. (Gesellschaft zur Förderung der Qualitatssicherung in medizinischen Laboratorien e.V.) using the primer set CLPS6. INSTAND e. V. is one of three reference institutions appointed by the German Medical Association and, thus, responsible for the organisation of EQAs for quality control in medical laboratories. Investigated "Ringversuch" samples consisted of a total of seven samples with different compositions (see table 4) and form the standard for the evaluation of the quality of a new SARS-CoV-2 detection method. The tests were carried out at the University Hospitals of Frankfurt and Münster and produced the following results:

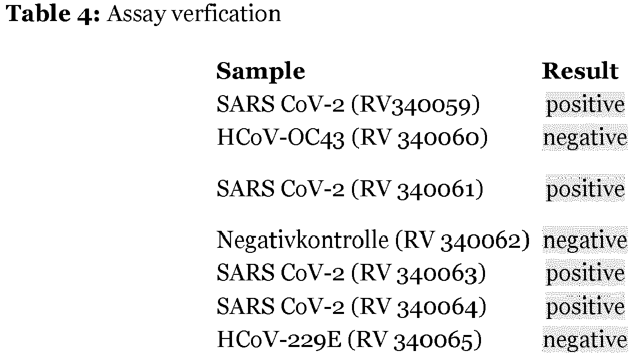

INSTAND e. V. is an interdisciplinary, not-for-profit, scientific medical society with over 300 members. It has been organising EQAs as part of external quality assurance in the area of haematology since 1968. In 1970, this service was extended to include nearly all areas of laboratory diagnostics. Envisaged goals include:

Optimal patient care through improved diagnostics, treatment monitoring, follow-up care and rehabilitation in the area of medicine
- Early detection of diseases by improving the quality of lab analyses and their assessment
- Promoting research on quality assurance in laboratory medicine
- Further training in all areas of medical laboratory diagnostics.

For more information: https://www.instand-ev.de/en/news/archives.html; e.g. Pre-evaluation of virology EQASs March 2016 https://www.instand-ev.de/fileadmin/uploads/Aktuelles/Pre-evaluation_Virology_March_2016_20160503b_EN.pdf and https://www.instand-ev.de/fileadmin/uploads/user upload/Dokumente/Virologie/20200214 INSTAND announce ment EQAS novel coronavirus SARS-CoV-2.pdf

### Example 3: Optimization of LAMP Primer sets

In order to demonstrate a further successful optimization of the LAMP primers according to the invention, in vitro transcribed SARS-CoV-2 RNA was diluted 10-fold serially and used as a template in RT-LAMP reactions using the 2×WarmStart Colorimetric from NEB, supplemented with guanidine hydrochloride, UTP and UDG. The final reaction volume was 10 µL, of which 3 µL contained varying amounts of viral RNA. Figure 9 shows the results for the optimized primer sets CLPS3 and CLPS4 compared to the old primer mix CLPS6. The inventors show that the optimization of the primer sets CLPS3 and CLPS4 significantly reduced the Ct value compared to the old primer mix CLPS6 (FIG. 9).

In addition, SARS-CoV-2 cell culture supernatant (Ct13; kindly provided by Frankfurt University Hospital) was used for RNA isolation. Extracted RNA was serially diluted 10-fold and used as a template in RT-LAMP reactions using NEB's 2×WarmSatrt Colorimetric, supplemented with guanidine hydrochloride, UTP and UDG. The final reaction volume was 10 µL, of which 3 µL contained varying amounts of viral RNA. Figure 10 shows the results for the published E and N primer sets of NEB (https://doi.org/10.1101/2020.06.03.132894) compared to the optimized new primer sets CLPS4 and CLPS5 of the present invention. The inventors show that the optimized primer sets CLPS4 and CLPS5 amplified lower virus copy numbers in the samples in a more stable manner than the E and N primer sets of NEB.

### Example 4: Evaluation of performance of optimized primer sets

Proof of performance of selected optimized primer sets CLPS1, CLPS2, CLPS3, CLPS4, and CLPS5: The previous experiment was continued for testing the primer mixes performance with different RNA concentrations. For this experiment SARS-CoV2 standard sample RV340059 was used as a template. The aim of this experiment was to determine and confirm the two optimal primer sets for use in the final version of the RT-LAMP assay according to the present invention.

Six different simplex reaction primer mixes CLPS1, CLPS2, CLPS3, CLPS4, and CLPS5, and ACTB primer set as control were prepared with each containing one of the six primer sets CLPS1, CLPS2, CLPS3, CLPS4, and CLPS5, and the control (ACTB) in question. The reaction volume was 25 µL in total with 5 µL being the template. A master mix for 10 samples for each of the SARS CoV-2 primer sets CLPS1, CLPS2, CLPS3, CLPS4, and CLPS5 and the control (ACTB) was prepared according to the following Table 5.

**Table 5: Master mix pipetting scheme for simplex LAMP.**

| Component | Volume |
|---|---|
| 2x Master Mix with UDG | 12.5 µL |
| SARS-CoV-2 Primer Mix | 1.25 µL |
| (F3/B3 stock solution: 2 µM | |
| FIP/BIP stock solution: 16 µM | |
| FL/BL stock solution: 8 µM) | |
| LAMP Fluorescent Dye (50× concentrate) | 0.25 µL |
| GnCl (1M) | 1 µL |
| MilliQ H2O | 5 µL |
| Template | 5 µL |
| Total volume | 25 µL |

The results of this primer test are shown in Figure 11 and 12. Fig. 11 shows the respective Ct values and Fig. 12 the respective Tm values of SARS CoV-2 primer test with SARS CoV2 standard sample RV340059 using six different primer dilutions ranging from undiluted to 1:1000.

It was observed that the undiluted SARS CoV2 standard sample RV340059 inhibited the reaction, which is due to the different sample matrix in which the SARS CoV-2 RNA was provided. This inhibition is no longer relevant once the sample was diluted 1:10. This dilution shows that primer sets CLPS3, CLPS2 and CLPS5 amplified the samples faster than primer sets CLPS1 and CLPS4, since their Ct values are lower. Regarding the results for the 1:100 dilution ratio, it can be seen that both primer mixes CLPS2 and CLPS4 performed best. Each of the five SARS CoV-2 primer mixes was only able to amplify the sample in one of the two replicates. Both primer sets CLPS1 and CLPS4 achieved the desired Ct value of 15 minutes or less. Since the overall performance of the primer mixes was too similar, more experiments were conducted to commit to a best performing final primer set for the RT-LAMP assay according to the present invention.

Confirmation of best performing primer sets on SARS CoV-2 supernatant dilutions: In addition, these results and the primer sets CLPS1 and CLPS2 selected for the final assay were to be confirmed in a further experiment.

The following material was provided:
- 1.5 mL tubes
- 8 well PCR Tube Stripes with attached flat caps
- CLPS1 Primer mix
- CLPS4 Primer mix
- ACTB Primer mix (Control)
- CLPS3 primer mix
- CLPS2 primer mix
- LAMP Fluorescent Dye (50× Concentrate) (NEB)
- WarmStart Colorimetric LAMP 2× Master Mix with UDG (NEB)
- Guanidine hydrochloride (40 mM)
- SB buffer
- milliQ H2O

Six different simplex reaction primer mixes were prepared with each containing one of the six primers in question. The reaction volume was 10 µL in total with 2 µL being the template. A master mix for 20 samples for each SARS CoV-2 primer mix was prepared according to the following Table 6.

**Table 6: Master mix pipetting scheme for simplex LAMP.**

| Component | Volume |
|---|---|
| 2x Master Mix with UDG | 5 µL |
| SARS-CoV-2 Primer Mix | 0.5 µL |
| (F3/B3 stock solution: 2 µM | |
| FIP/BIP stock solution: 16 µM | |
| FL/BL stock solution: 8 µM) | |
| LAMP Fluorescent Dye (50× concentrate) | 0.1 µL |
| GnCl (1M) | 0.4 µL |
| MilliQ H2O | 2 µL |
| Template | 2 µL |
| Total volume | 10 µL |

Subsequently, the master mixes were aliquoted into 8-well PCR tube strips with attached flat caps with a volume of 8 µL master mix for each reaction.

Eight different dilutions were used in the experiments each tested in duplicates. A serial dilution was prepared in an 8-well PCR tube strip with attached flat caps. For this, 25 µL of clinical samples provided from University Hospital, Frankfurt am Main (UKF) were pipetted into the first well and mixed with 75 µL GPS. To prepare the dilution matrix 900 µL gargle sample (G) were mixed with 100 µL 10× stabilization buffer (GPS). For the dilutions used in the experiment GPS was spiked in a different ratio with clinical samples accordingly to Table 7.

**The following Table 7 shows the pipetting scheme for dilutions.**

| Dilution | clinical samples | GPS |
|---|---|---|
| 1:5 | 10 µL | 40 µL |
| 1:10 | 5 µL | 45 µL |
| 1:20 | 2.5 µL | 47.5 µL |
| Negative control (NC) | -- | 50 µL |

50 µL of this 1:4 dilution was then pipetted into the second well and mixed with 50 µL GPS. This was repeated for the entire strip, resulting in eight different dilutions ranging in from 1:4 to 1:512. The samples were than inactivated at 95°C for 10 minutes. After inactivation and cooling, the samples were added directly to the master mix in another laboratory area, vortexed and spun down. Samples were then placed in Analytik Jena qTower 2.2 and processed according to Table 8.

**Table 8: Isothermal amplification program.**

| Step | Temperature | Duration | Repetition |
|---|---|---|---|
| Incubation | 25°C | 5 min | - |
| Incubation | 65°C | 1 min | 45x |
| Melting curve | 75°C-95°C | - | - |

The results of testing the best performing primer sets on SARS CoV-2 supernatant dilutions are shown in Figure 13 and 14. Fig. 13 shows the Ct values and Fig. 14 Tm values of SARS CoV-2 primer mix comparison using six different primers in serial dilution of clinical samples in GPS. Dilutions ranging from 1:4 to 1:512.

In summary, of the five SARS CoV-2 primer sets tested, the CLPS1, CLPS3 and CLPS2 primer sets performed best as described above. Therefore, the primer sets CLPS4 and CLPS5 were no longer considered for the final assay protocol. Since the CLPS3 and CLPS2 primer mixes are too similar in performance, both sets of primers were used in the following experiments. Since the CLPS1 primer mix is the only primer mix that detected the sample at a dilution ratio of 1:512, the high sensitivity is a decisive argument for selecting the CLPS1 primer mix as one of the final primer sets for the RT-LAMP assay in accordance with the present invention. Furthermore, the CLPS1 primer set stands out as reliable especially with regard to clinical samples.

Verification of selected, best performing primer sets on serial dilution of reference samples: For this experiment, the three different primer sets CLPS1, CLPS3 and CLPS2 with the best performance shown were used. The experiments were each carried out in a duplex reaction with ATCB primer mix. The reaction volume of each sample was 25 µL in total, with 5 µL serving as template. A master mix for 38 samples for each SARS CoV-2 primer mix was prepared according to Table 9 below.

**Table 9: Master mix pipetting scheme for duplex LAMP.**

| Component | Volume |
|---|---|
| 2x Master Mix with UDG | 12.5 µL |
| SARS-CoV-2 Primer Mix | 1.25 µL |
| ACTB primer mix | 0.5 µL |
| LAMP Fluorescent Dye | 0.25 µL |
| GnCl (1M) | 1 µL |
| MilliQ H2O | 4.5 µL |
| Template | 5 µL |
| Total volume | 25 µL |

The master mixes were aliquoted into 8-well PCR tube strips with attached flat caps with a volume of 20 µL master mix for each reaction.

Serial dilutions of reference sample 1 and 2 were prepared in one 8-well strip each. 100 µL of each dilution was prepared. It was started with 100 µL of the undiluted sample in the first well. In the second well, 100 µL of the undiluted sample were added to 100 µL of GPS, resulting in a 1:2 dilution ratio, with the GPS consisting of a 1:10 ratio of gargle solution (G) spiked with stabilizing buffer. Subsequently, 100 µL of the 1:2 dilutions were added to 100 µL of GPS in the third well, giving a dilution ratio of 1:4. This procedure was repeated for the entire strip. This resulted in eight different dilutions for each reference sample. Table 10 shows the respective serial dilutions. Each dilution was tested in duplicates.

**Table 10: serial dilution of reference sample**

| | | | | | | | |
|---|---|---|---|---|---|---|---|
| undiluted | 1:2 | 1:4 | 1:8 | 1:16 | 1:32 | 1:64 | 1:128 |

The samples were then inactivated at 95°C for 10 minutes. After inactivation and cooling, the samples were added directly to the master mix in another laboratory area, vortexed and spun down. Then, the samples were placed in the Analytik Jena qTower 2.2 and processed according to Table 11.

**Table 11: Isothermal amplification program.**

| Step | Temperature | Duration | Repetition |
|---|---|---|---|
| Incubation | 25°C | 5 min | - |
| Incubation | 65°C | 1 min | 30x |
| Melting curve | 80°C-96°C | - | - |

After completion of the amplification, lateral flow dipsticks were performed on some samples for further analysis. One 1:4 duplicate per reference sample and primer mix was tested, as well as one of the 1:2 dilutions for reference sample for primer sets CLPS2 and CLPS3.

Figure 15 and 16 shows the results of the experiment. Fig. 15 shows the Ct values and Fig. 16 shows Tm values of SARS CoV-2 reference samples 1 and 2 in serial dilutions from undiluted to 1:128 by using the three selected primer sets CLPS1, CLPS3 and CLPS2 each in a duplex reaction with control (ACTB).

All primer mixes reliably detected reference sample 1 (10,000,000 copies/ml), the only problem being that the undiluted sample was too strong so that actin could not be amplified. This could also be due to the matrix of the undiluted sample inhibiting the LAMP reaction, as can be presumed from the higher Ct values of the undiluted samples compared to the diluted samples.

The same problem also occurred with the undiluted samples of reference sample 2 (1,000,000 copies/ml). One out of two replicates for CLPS1 and CLPS2 did not amplify at all, which is another indication that the sample matrix interferes with the LAMP reaction. The CLPS1 primer mix reliably recognized reference sample 2 in both replicates up to a dilution ratio of 1:16. At dilutions of 1:32 to 1:128, SARS CoV-2 RNA could be detected in one of each of two replicates. The CLPS3 primer mix could reliably detect reference sample 2 up to a dilution ratio of 1:32. Of the three SARS CoV-2 primer mixes, CLPS2 performed best. The CLPS2 primer mix showed the same problems as the undiluted reference sample 2 and was able to reliably detect the sample in all dilutions, with both replicates being positive for SARS CoV-2 and actin.

This experiment is the first argument for choosing primer set CLPS2 over CLPS3 for the final RT-LAMP assay according to the present invention, as both target a similar region of the same gene. The other primer considered for the final assay protocol would be CLPS1, as it has shown reliable performance a number of experiments as it is described above and its target region does not overlap with either primer set CLPS2 or CLPS3.

### Example 5: Triplex LAMP experiments

Determination of the primer concentration to be used: Four different primer concentrations for the SARS-CoV-2 specific primer sets were tested in triplex with control (ACTB) (constant concentration ACTB primer with 0.5 µL). The results are shown in Figure 17 and Table 12. Table 12 below shows the Ct values of diluted reference sample 1 from 1000 copy/µL to 31.25 copy/µL.

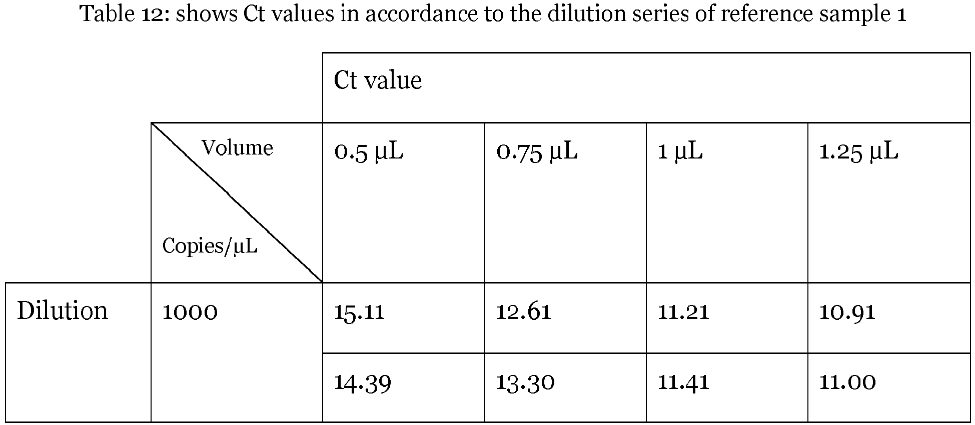

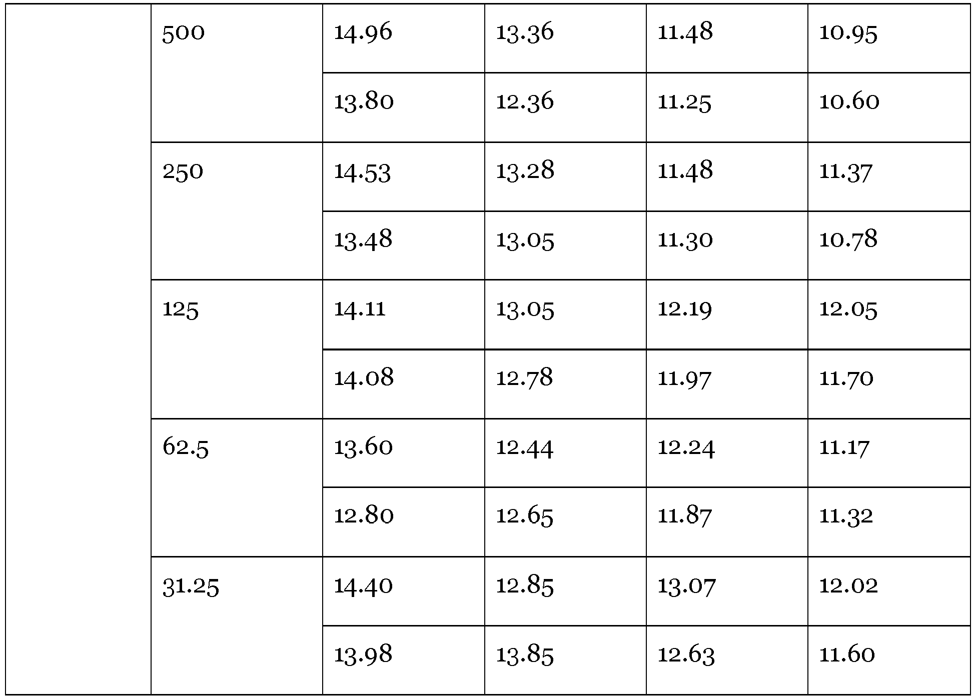

All samples were positive for SARS-CoV-2, but identifiable differences and trend in Ct values were noticed. Fortunately, the measured Ct values were higher than desired at lower concentrations of the primers.

Comparison of Triplex-LAMP and Duplex-LAMP: In a further experiment, Triplex-LAMP was compared with Duplex-LAMP. Therefore, three different primer concentrations for the SARS-CoV-2 specific primers in triplex with control (ACTB) (constant concentration ACTB primer with 0.5 µL) were compared with the duplex reactions CLPS2/ control (ACTB) and CLPS1/ control (ACTB). Figure 17 and Table 13 show the results. Table 13 below shows the Ct values of Triplex-LAMP and Duplex-LAMP of diluted reference sample 1 from 1000 copy/µL to 31.25 copy/µL.

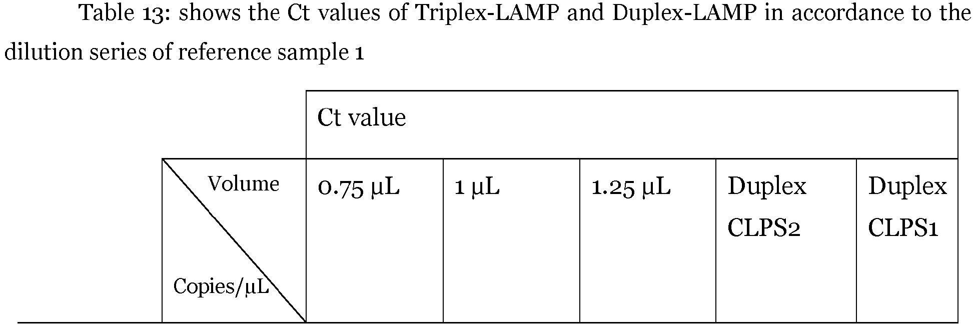

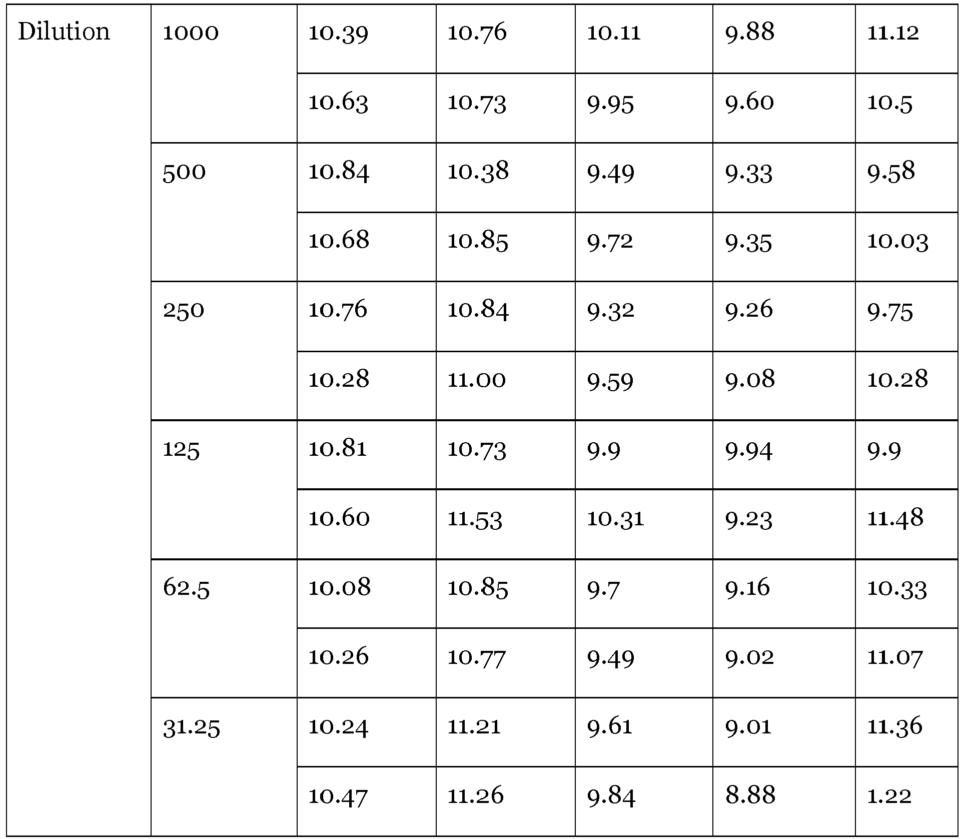

The Ct values of the Duplex LAMP are comparable to the Ct values of the Triplex LAMP. SARS CoV-2 was indicated in all samples.

Determination of the best control (ACTB) primer concentration: Another experiment was carried out to determine the best control (ACTB) primer concentration in the triplex. The results are shown in Figure 19.

Three different primer concentrations were tested for the control (ACTB) (F3_{/}B3 stock solution: 2 µM; FIP/BIP stock solution: 16 µL; FL/BL stock solution: 8 µM/0.5 µL, 0.4 µL, 0.3 µL).

As shown in Fig. 19, the Ct values are lowest for an control (ACTB) concentration of 0.5 µL. It is therefore the favored concentration for the triplex reaction, wherein the F3_{/}B3 primer solution has a concentration of 100 nM; the FIP/BIP primer solution has a concentration of 800 nM and the FL/BL primer solution has a concentration of 400 nM for 0.5µL in 10µL (final).

### Example 6: Analysis of SARS CoV-2 human infected patient samples

The specificity and sensitivity of the primer sets according to the invention were demonstrated under clinical conditions. Clinical samples from human SARS CoV-2 infected patients were provided from the University Hospital Münster (UKM) and the University Hospital, Frankfurt am Main (UKF).

Therefore, SARS CoV-2 was first determined in throat swab samples derived from human patients infected with SARS CoV-2: The SARS CoV-2 RT-LAMP according to the methods of the present invention was performed on purified RNA from patient samples. The samples all derived from throat swabs from human patients infected with SARS CoV-2. Then, 3 µL of purified RNA of each sample was added in 7 µL Master Mix in duplicates. WarmStart^{®} Colorimetric LAMP 2X Master Mix (DNA & RNA), 225 mM Betaine; UTP/UDG primer set CLPS6.

Then, the reaction mixture was incubated at 25°C for 5 minutes. The temperature was increased up to 65°C and the reaction mixture was incubated for 45 minutes.

Subsequently, Ct values were determined. In the following Table 14 represents the results of the analysis of SARS CoV-2 infected human patient samples.

**Table 14: shows the results of RT-LAMP for determining SARS CoV-2 in clinical samples using the primer sets according to the invention.**

| Sample | Mean Cₜ value [min] | Cₜ value [min] | Results LAMP |
|---|---|---|---|
| Sample 1 | 16 | 15.18 | positive |
| | | 14.05 | positive |
| Sample 2 | 23,5 | 19.46 | positive |
| | | 18.89 | positive |
| Sample 3 | 27 | 20.05 | positive |
| | | 21.48 | positive |
| Sample 4 | 28 | 20.83 | positive |
| | | 18.46 | positive |
| Sample 5 | 37 | 33.06 | positive |
| | | No Cₜ | negative |
| Sample 6 | No Cₜ | No Cₜ | negative |
| | | No Cₜ | negative |

In a further experiment, SARS CoV-2 in gargle samples (G) was compared with throat swab samples (T) derived from human patients infected with SARS CoV-2: Analysis of SARS CoV-2 infected patient samples: SARS CoV-2 RT-LAMP was performed on unpurified patient samples from gargles (G) and throat swabs (T); First, the samples were heat-inactivated for 2 minutes at 95°C. Subsequently, 3 µL of each sample was pipetted into 7 µL master mix (WarmStart^{®} LAMP Kit (DNA & RNA)) with 200 mM betaine; dUTP/UDG; and primer set CLPS6. Then, the reaction mixtures were incubated at 25°C for 5 minutes, and afterwards at 65°C for 45 minutes.

Subsequently, Ct values were determined. In the following Table 15 represents the results of the comparison of SARS CoV-2 in gargle samples (G) with throat swab samples (T) derived from human patients infected with SARS CoV-2.

**Table 15: Ct values of SARS CoV-2 infected patient samples**

| Sample* | | Cₜ value [min] | Results LAMP |
|---|---|---|---|
| Sample 7 | G: Cₜ 38.76 | No Cₜ | negative |
| | T: Cₜ 39.39 | No Cₜ | negative |
| Sample 8 | G: Cₜ 31.80 | 21.47 | positive |
| | T: Cₜ 32.15 | No Cₜ | negative |
| Sample 9 | G: Cₜ 39.71 | No Cₜ | negative |
| | T: Cₜ 39.72 | No Cₜ | negative |
| Sample 10 | G: No Cₜ | No Cₜ | negative |
| | T: No Cₜ | No Cₜ | negative |
| Sample 11 | G: Cₜ 24.32 | 17.74 | positive |
| | T: Cₜ 24.45 | 21.54 | positive |

| | | | |
|---|---|---|---|
| *Reference Cₜ values to purified RNA from gargle water (G) and throat swab (T), standard RT-PCR. | | | |

**Example 7:** Proof of performance of selected primer sets on ring test samples and cell culture supernatant of clinical samples

All experiments were performed with the current assay on unpurified samples diluted 1:4 in gargle sample (G) matrix with stabilization buffer.

First, the specificity was tested. Table16 shows the results of the LAMP assay by using the selected primer sets CLPS1 and CLPS2.

**Table 16: Ct values of ring test sample by using selected primer sets CLPS1 and CLPS2.**

| Ring test sample | | Concentration dilution | CLPS1 | CLPS2 |
|---|---|---|---|---|
| MRC-5 cells | RV340070 | 1:6 | No Cₜ | No Cₜ |
| MERS CoV | RV340067 | 1:1,000 | No Cₜ | No Cₜ |
| HCoV 229E | RV340068 | 1:1,000 | No Cₜ | No Cₜ |
| HCoV NL63 | RV340072 | 1:1,000 | No Cₜ | No Cₜ |
| HCoV OC43 | RV340074 | 1:1,000 | No Cₜ | No Cₜ |

Fortunately, no cross-reactivity with other coronaviruses from ring tests in all replicates was indicated.

Further ring test samples were selected to continue the experiments for the proof of performance of the selected primer sets.

Therefore, unpurified samples were diluted 1:4 in gargle sample (G) with SB buffer (GPS). Following steps were performed:Heat inactivation of samples for 10 minutes at 95°C. Then, 5 µL sample were pipetted into 20 µL Master Mix. WarmStart^{®} Colorimetric LAMP Kit with UDG were used. 40 mM guanidine hydrochloride was added. SARS CoV-2 primer mix CLPS1 and CLPS2 were added together with the control (ACTB). The reaction mixtures were incubated at 25°C for 5 minutes, and subsequently at 65°C for 25 minutes. Table 17 shows the results of the LAMP assay by using the selected primer sets CLPS1 and CLPS2.

**Table 17: Ct values of ring test samples by using selected primer sets CLPS1 and CLPS2.**

| Dilution series reference sample 1 | | |
|---|---|---|
| Copies/µL | CLPS1 | CLPS2 |
| 1,000 | 8.8 | 8.2 |
| | 9.0 | 8.6 |
| 100 | 10.4 | 9.6 |
| | 9.6 | 9.4 |
| 10 | 9.4 | 10.3 |
| | 10.4 | 9.6 |
| 1 | 10.6 | 10.5 |
| | 9.8 | 9.9 |
| 0.1 | 10.2 | No Cₜ |
| | 10.6 | 9.7 |
| 0.01 | No Cₜ | No Cₜ |
| | No Cₜ | No Cₜ |
| 0.001 | No Cₜ | No Cₜ |
| | No Cₜ | No Cₜ |

SARS CoV-2 was reliably detected in the respective diluted reference sample 1.

**Table 19: shows the Ct values of diluted SARS CoV-2 cell culture supernatant samples of clinical samples by using selected primer sets CLPS1 and CLPS2.**

| Dilution series SARS CoV-2 cell culture supernatant | | |
|---|---|---|
| Dilution | CLPS1 | CLPS2 |
| 1:4 | 9.9 | 9.6 |
| | 9.2 | 9.4 |
| 1:8 | 9.1 | 9.7 |
| | 9.7 | 8.9 |
| 1:16 | 9.3 | 10.4 |
| | 10.1 | 9.2 |
| 1:32 | 9.1 | 10.1 |
| | 10.7 | 9.1 |
| 1:64 | 9.3 | 9.9 |
| | 10.6 | 9.8 |
| 1:128 | 13.0 | 13.3 |
| | No Cₜ | No Cₜ |
| 1:256 | No Cₜ | 13.6 |
| | 15.2 | No Cₜ |
| 1:512 | 15.7 | No Cₜ |
| | No Cₜ | No Cₜ |

The inventors are fortunately able to demonstrate with this experiment that the preferred primer sets (CLPS1 and CLPS2) of the present invention reliably detect SARS CoV-2 in cell culture supernatants from the Instand reference sample and the clinical samples with high sensitivity. Furthermore, SARS CoV-2 was shown to be detectable in the reference sample to 1 copy/µL. It is noted, that primer set CLPS1 is partially more sensitive for cell culture supernatants from clinical samples.

### Example 8: Melting curve analysis

Melting curve analysis of SARS CoV-2 LAMP was performed. The results are shown in Figure 20. Therefore, cell culture supernatant from clinical samples near limit of detection (LOD) in stabilized gargle sample (GPS) was provided and the following steps were performed:
- Heat inactivation of samples 10 minutes at 95°C
- 5 µL sample were pipetted into 20 µL Master Mix
- WarmStart^{®} Colorimetric LAMP Kit with UDG were used for the performance of the method
- 40 mM guanidine hydrochloride was added into the reaction mixture
- Selected primer Mix CLPS2 was mixed together with the control (ACTB) in different concentrations: 0.6X, 0.4X and 0.2X
- The reaction mixtures were incubated at 25°C for 5 minutes, and afterwards at 65°C, for further 25 minutes

Figure 20 shows the results of the melting curve analysis; With a percentage of 40% of the control (ACTB) optimal results for stable actin amplification were repeatedly shown and furthermore SARS CoV-2 was detected in weak samples.

### Example 9: Proof of sample stability stabilized with stabilization buffer

In a final experiment the sample stability was investigated, when samples are stored in stabilization buffer (SB). Therefore, the following steps were performed:
- Instand ring test sample (SARS CoV2 standard sample RV340059) dilution series was provided with/ and without SB
- Heat inactivation of samples for 10 minutes at 95°C after 0, 1 h and 2 h
- 5 µL sample were pipetted into 20 µL Master Mix
- WarmStart^{®} Colorimetric LAMP Kit with UDG were used for the performance of the method
- 40 mM guanidine hydrochloride was added into each sample
- Selected primer set CLPS2 was mixed together with the control (ACTB)
- The reaction mixtures were incubated at 25°C for 5 minutes, and afterwards at 65°C for further 25 minutes

**Table 20: shows Ct values of samples with and without SB measured at different time points.**

| Buffer | Dilution | Measuring time after sample preparation | | | | | |
|---|---|---|---|---|---|---|---|
| | | o h | | 1 h | | 2 h | |
| Without SB | 1:10 | 10.0 | 9.9 | 10.3 | 9.3 | No Cₜ | No Cₜ |
| | 1:100 | No Cₜ | No Cₜ | No Cₜ | No Cₜ | No Cₜ | No Cₜ |
| | 1:1,000 | No Cₜ | No Cₜ | No Cₜ | No Cₜ | No Cₜ | No Cₜ |
| With SB | 1:10 | 7.9 | 7.9 | 7.7 | 7.9 | 7.6 | 8.0 |
| | 1:100 | 8.7 | 9.0 | 9.6 | 8.4 | 9.5 | 8.8 |
| | 1:1,000 | 9.3 | No Cₜ | No Cₜ | 8.8 | 9.2 | No Cₜ |

In view of the results, it was shown that SB buffer stabilizes the samples during inactivation and post inactivation. Furthermore, results of an initial time course study on sample integrity indicate that SB also increases RNA quality (and thereby its stability) of inactivated samples at RT and at a temperature of 4°C for at least 3 days (reference sample 1,000 copies/µL).

## Claims

1. A set of nucleic acid primers for use in Loop-Mediated Isothermal Amplification (LAMP) of a Severe Acute Respiratory Syndrome-Corona Virus 2 (SARS-Cov2) derived target nucleic acid, comprising at least a first Corona LAMP Primer Set, wherein the first Corona LAMP Primer Set comprises the following nucleic acid primer molecules:
(i) A nucleic acid F3 LAMP primer which (i) comprises, or consists essentially of, a sequence shown in SEQ ID NO: 47 (GGACCCCAAAATCAG), or a sequence having no more than 5, or no more than 3, preferably no more than 1 nucleotide alterations therefrom selected from addition(s), deletion(s), and substitution(s), or (ii) hybridizes under stringent conditions to a target nucleic sequence which is complementary to the sequence shown in SEQ ID NO: 47 (GGACCCCAAAATCAG); and
(ii) A nucleic acid FL LAMP primer which (i) comprises, or consists essentially of, a sequence shown in SEQ ID NO: 94 (GTTGAATCTGAGGGTCC), or a sequence having no more than 5, or no more than 3, preferably no more than 1 nucleotide alterations therefrom selected from addition(s), deletion(s), and substitution(s), or (ii) hybridizes under stringent conditions to a target nucleic sequence which is complementary to the sequence shown in SEQ ID NO: 94 (GTTGAATCTGAGGGTCC); and
(iii) A nucleic acid FIP LAMP primer which (i) comprises, or consists essentially of, a sequence shown in SEQ ID NO: 95 (TCTCCATTCTGGTTACTGCCCGAAATGCACCCCGCATTAC), or a sequence having no more than 5, or no more than 3, preferably no more than 1 nucleotide alterations therefrom selected from addition(s), deletion(s), and substitution(s), or (ii) hybridizes under stringent conditions to a target nucleic sequence which is complementary to the sequence shown in SEQ ID NO: 95 (TCTCCATTCTGGTTACTGCCCGAAATGCACCCCGCATTAC); and
(iv) A nucleic acid B₃ LAMP primer which (i) comprises, or consists essentially of, a sequence shown in SEQ ID NO: 56 (CTTGCCATGTTGAGTG), or a sequence having no more than 5, or no more than 3, preferably no more than 1 nucleotide alterations therefrom selected from addition(s), deletion(s), and substitution(s), or (ii) hybridizes under stringent conditions to a target nucleic sequence which is complementary to the sequence shown in SEQ ID NO: 56 (CTTGCCATGTTGAGTG); and
(v) A nucleic acid BL LAMP primer which (i) comprises, or consists essentially of, a sequence shown in SEQ ID NO: 53 (CCCCAAGGTTTACCC), or a sequence having no more than 5, or no more than 3, preferably no more than 1 nucleotide alterations therefrom selected from addition(s), deletion(s), and substitution(s), or (ii) hybridizes under stringent conditions to a target nucleic sequence which is complementary to the sequence shown in SEQ ID NO: 53 (CCCCAAGGTTTACCC); and
(vi) A nucleic acid BIP LAMP primer which (i) comprises, or consists essentially of, a sequence shown in SEQ ID NO: 55 (CGCGATCAAAACAACGTCGGAGCGGTGAACCAAGACGCAG), or a sequence having no more than 5, or no more than 3, preferably no more than 1 nucleotide alterations therefrom selected from addition(s), deletion(s), and substitution(s), or (ii) hybridizes under stringent conditions to a target nucleic sequence which is complementary to the sequence shown in SEQ ID NO: 55 (CGCGATCAAAACAACGTCGGAGCGGTGAACCAAGACGCAG).

2. A set of nucleic acid primers for use in Loop-Mediated Isothermal Amplification (LAMP) of a Severe Acute Respiratory Syndrome-Corona Virus 2 (SARS-Cov2) derived target nucleic acid, comprising at least a second Corona LAMP Primer Set, wherein the second Corona LAMP Primer Set comprises the following nucleic acid primer molecules:
(i) A nucleic acid F3 LAMP primer which (i) comprises, or consists essentially of, a sequence shown in SEQ ID NO: 1 (ATGACCAAATTGGCTACTAC), or a sequence having no more than 5, or no more than 3, preferably no more than 1 nucleotide alterations therefrom selected from addition(s), deletion(s), and substitution(s), or (ii) hybridizes under stringent conditions to a target nucleic sequence which is complementary to the sequence shown in SEQ ID NO: 1 (ATGACCAAATTGGCTACTAC); and
(ii) A nucleic acid FL LAMP primer which (i) comprises, or consists essentially of, a sequence shown in SEQ ID NO: 12 (CCATCTTGGACTGAG), or a sequence having no more than 5, or no more than 3, preferably no more than 1 nucleotide alterations therefrom selected from addition(s), deletion(s), and substitution(s), or (ii) hybridizes under stringent conditions to a target nucleic sequence which is complementary to the sequence shown in SEQ ID NO: 12 (CCATCTTGGACTGAG); and
(iii) A nucleic acid FIP LAMP primer which (i) comprises, or consists essentially of, a sequence shown in SEQ ID NO: 4 (AGCTTCTGGCCCAGTTCCTTCGTGGTGGTGACGG), or a sequence having no more than 5, or no more than 3, preferably no more than 1 nucleotide alterations therefrom selected from addition(s), deletion(s), and substitution(s), or (ii) hybridizes under stringent conditions to a target nucleic sequence which is complementary to the sequence shown in SEQ ID NO: 4 (AGCTTCTGGCCCAGTTCCTTCGTGGTGGTGACGG); and
(iv) A nucleic acid B3 LAMP primer which (i) comprises, or consists essentially of, a sequence shown in SEQ ID NO: 68 (AGCACGATTGCAGCAT), or a sequence having no more than 5, or no more than 3, preferably no more than 1 nucleotide alterations therefrom selected from addition(s), deletion(s), and substitution(s), or (ii) hybridizes under stringent conditions to a target nucleic sequence which is complementary to the sequence shown in SEQ ID NO: 68 (AGCACGATTGCAGCAT); and
(v) A nucleic acid BL LAMP primer which (i) comprises, or consists essentially of, a sequence shown in SEQ ID NO: 65 (ACTGAGGGAGCCTTGA), or a sequence having no more than 5, or no more than 3, preferably no more than 1 nucleotide alterations therefrom selected from addition(s), deletion(s), and substitution(s), or (ii) hybridizes under stringent conditions to a target nucleic sequence which is complementary to the sequence shown in SEQ ID NO: 65 (ACTGAGGGAGCCTTGA); and
(vi) A nucleic acid BIP LAMP primer which (i) comprises, or consists essentially of, a sequence shown in SEQ ID NO: 67 (CAAAGACGGCATCATATGGGGGATTGCGGGTGCCAATGTG), or a sequence having no more than 5, or no more than 3, preferably no more than 1 nucleotide alterations therefrom selected from addition(s), deletion(s), and substitution(s), or (ii) hybridizes under stringent conditions to a target nucleic sequence which is complementary to the sequence shown in SEQ ID NO: 67 (CAAAGACGGCATCATATGGGGGATTGCGGGTGCCAATGTG).

3. The set of nucleic acid primers of claim 1 or 2, comprising both the first Corona LAMP Primer Set recited in claim 1 and the second Corona LAMP Primer Set recited in claim 2.

4. The set of nucleic acid primers of any one of claims 1 to 3, wherein the F3 LAMP primer, the FL LAMP Primer, B3 LAMP primer, and/or the BL LAMP Primer each have, or consist of, a length of 5 to 30 nucleotides (nt), preferably of 8 to 25 nt.

5. The set of nucleic acid primers of any one of claims 1 to 4, wherein the FIP LAMP primer, and/or the BIP LAMP Primer each have, or consist of, a length of 10 to 50 nucleotides (nt), preferably of 15 to 40 nt.

6. The set of nucleic acid primers of any one of claims 1 to 5, wherein at least one primer in the set, preferably more than one primer in the set, comprises a detectable label, such as a detectable label selected from biotin, FAM, or DIG.

7. The set of nucleic acid primers of any one of claims 1 to 6, wherein the LAMP is reverse transcription LAMP (RT-LAMP).

8. A container package, comprising at least a first container, wherein the first container comprises a primer mix composition of all nucleic acid primer molecules of the first Corona LAMP Primer Set recited in claim 1.

9. A container package comprising at least a second container, wherein the second container comprises a primer mix composition of all nucleic acid primer molecules of the second Corona LAMP Primer Set recited in claim 2.

10. The container package of claim 8 or 9, comprising both the first container recited in claim 8 and the second container recited in claim 9.

11. An *in-vitro* method of detecting presence of a SARS CoV2 nucleic acid in a sample, comprising:
(i) Contacting the sample with at least a first Corona LAMP Primer Set and/or a second Corona LAMP Primer Set as recited in any one of claims 1 to 7 under conditions sufficient for amplification of the corona virus nucleic acid, thereby producing a SARS CoV2 nucleic acid amplification product; and
(ii) Detecting the corona virus nucleic acid amplification product and thereby detecting the presence of the SARS CoV2 nucleic acid in the sample.

12. The method of claim 11, further comprising contacting the sample with a reverse transcriptase under conditions sufficient for reverse transcription of the corona virus nucleic acid.

13. The method of claim 11 or 12, wherein detecting the corona virus nucleic acid amplification product comprises turbidity measurement, (real-time) fluorescence detection, visual detection/colorimetric analysis, lateral flow immunochromatographic assays or gel electrophoresis.

14. The method of any one of claims 11 to 13, wherein the sample comprises isolated DNA, isolated RNA, preferably wherein the sample is a respiratory tract sample and most preferably is a gurgle sample or a nose/throat swap.

15. The method of any one of claims 11 to 14, wherein in (a) one sample is contacted with least the first Corona LAMP Primer Set and the second Corona LAMP Primer set in one single amplification reaction.

16. A diagnostic kit, comprising a container package of any one of claim 8 to 10, and at least one component necessary for conducting a RT-LAMP.

17. A use of a set of nucleic acid primers of any one of claims 1 to 7 in the diagnosis of a SARS-CoV2 infection in a mammalian, preferably human, subject.


SEQUENCE LISTING

<110>  Fraunhofer-Gesellschaft zur Foerderung der angewandten
       Forschung e.V.

<120>  METHODS AND MEANS FOR CORONA VIRUS NUCLEIC ACID DETECTION

<130>  F31479EP01

<150>  EP20182595.7
<151>  2020-06-26

<160>  95

<170>  PatentIn version 3.5

<210>  1
<211>  20
<212>  DNA
<213>  artificial

<220>
<223>  LAMP  probe or primer

<400>  1
atgaccaaat tggctactac                                                  20


<210>  2
<211>  16
<212>  DNA
<213>  artificial

<220>
<223>  LAMP  probe or primer

<400>  2
ttcgtggtgg tgacgg                                                      16


<210>  3
<211>  18
<212>  DNA
<213>  artificial

<220>
<223>  LAMP  probe or primer

<400>  3
agcttctggc ccagttcc                                                    18


<210>  4
<211>  34
<212>  DNA
<213>  artificial

<220>
<223>  LAMP  probe or primer

<400>  4
agcttctggc ccagttcctt cgtggtggtg acgg                                  34

<210>  5
<211>  34
<212>  DNA
<213>  artificial

<220>
<223>  LAMP  probe or primer


<220>
<221>  BIOTIN
<222>  (1)..(1)
<223>  Biotin Label

<400>  5
agcttctggc ccagttcctt cgtggtggtg acgg                                    34


<210>  6
<211>  34
<212>  DNA
<213>  artificial

<220>
<223>  LAMP  probe or primer


<220>
<221>  JOE
<222>  (1)..(1)
<223>  JOE label

<400>  6
agcttctggc ccagttcctt cgtggtggtg acgg                                    34


<210>  7
<211>  17
<212>  DNA
<213>  artificial

<220>
<223>  LAMP  probe or primer

<400>  7
attggcaccc gcaatcc                                                       17


<210>  8
<211>  16
<212>  DNA
<213>  artificial

<220>
<223>  LAMP  probe or primer

<400>  8
gaggaagttg tagcac                                                        16


<210>  9
<211>  17

<212>  DNA
<213>  artificial

<220>
<223>  LAMP  probe or primer

<400>  9
cgagaagagg cttgact                                                    17


<210>  10
<211>  33
<212>  DNA
<213>  artificial

<220>
<223>  LAMP  probe or primer

<400>  10
attggcaccc gcaatccgag gaagttgtag cac                                  33


<210>  11
<211>  33
<212>  DNA
<213>  artificial

<220>
<223>  LAMP  probe or primer


<220>
<221>  DIG
<222>  (1)..(1)
<223>  DIG Label

<400>  11
attggcaccc gcaatccgag gaagttgtag cac                                  33


<210>  12
<211>  15
<212>  DNA
<213>  artificial

<220>
<223>  LAMP  probe or primer

<400>  12
ccatcttgga ctgag                                                      15


<210>  13
<211>  15
<212>  DNA
<213>  artificial

<220>
<223>  LAMP  probe or primer

<400>  13
ccatcttgga ctgag                                                      15

<210>  14
<211>  15
<212>  DNA
<213>  artificial

<220>
<223>  LAMP  probe or primer


<220>
<221>  FAM
<222>  (1)..(1)
<223>  FAM Label

<400>  14
tgctaacaat gctgc                                                        15


<210>  15
<211>  17
<212>  DNA
<213>  artificial

<220>
<223>  LAMP  probe or primer

<400>  15
attggcaccc gcaatcc                                                      17


<210>  16
<211>  20
<212>  DNA
<213>  artificial

<220>
<223>  LAMP  probe or primer

<400>  16
cgtagaagcc ttttggcaat                                                   20


<210>  17
<211>  37
<212>  DNA
<213>  artificial

<220>
<223>  LAMP  probe or primer

<400>  17
attggcaccc gcaatcccgt agaagccttt tggcaat                                37


<210>  18
<211>  16
<212>  DNA
<213>  artificial

<220>
<223>  LAMP  probe or primer

<400>  18
gtgctacaac ttcctc                                                        16


<210>  19
<211>  22
<212>  DNA
<213>  artificial

<220>
<223>  LAMP  probe or primer

<400>  19
atggcacttg tggcttagta ga                                                 22


<210>  20
<211>  18
<212>  DNA
<213>  artificial

<220>
<223>  LAMP  probe or primer

<400>  20
cttgaacagc cctatgtg                                                      18


<210>  21
<211>  20
<212>  DNA
<213>  artificial

<220>
<223>  LAMP  probe or primer

<400>  21
ctacgaccgt actgaatgcc                                                    20


<210>  22
<211>  38
<212>  DNA
<213>  artificial

<220>
<223>  LAMP  probe or primer

<400>  22
ctacgaccgt actgaatgcc cttgaacagc cctatgtg                                38


<210>  23
<211>  38
<212>  DNA
<213>  artificial

<220>
<223>  LAMP  probe or primer


<220>
<221>  DIG

<222>  (1)..(1)
<223>  DIG Label

<400>  23
ctacgaccgt actgaatgcc cttgaacagc cctatgtg                              38


<210>  24
<211>  16
<212>  DNA
<213>  artificial

<220>
<223>  LAMP  probe or primer

<400>  24
cctcatgtgg gcgaaa                                                      16


<210>  25
<211>  24
<212>  DNA
<213>  artificial

<220>
<223>  LAMP  probe or primer

<400>  25
cctaagtcaa atgactttag atcg                                             24


<210>  26
<211>  21
<212>  DNA
<213>  artificial

<220>
<223>  LAMP  probe or primer

<400>  26
agctcacgca tgagttcacg g                                                21


<210>  27
<211>  40
<212>  DNA
<213>  artificial

<220>
<223>  LAMP  probe or primer

<400>  27
cctcatgtgg gcgaaaccta agtcaaatga ctttagatcg                            40


<210>  28
<211>  18
<212>  DNA
<213>  artificial

<220>
<223>  LAMP  probe or primer

<400>  28
ttctgctacc agctcaac                                                     18


<210>  29
<211>  18
<212>  DNA
<213>  artificial

<220>
<223>  LAMP  probe or primer


<220>
<221>  FAM
<222>  (1)..(1)
<223>  FAM Label

<400>  29
ttctgctacc agctcaac                                                     18


<210>  30
<211>  20
<212>  DNA
<213>  artificial

<220>
<223>  LAMP  probe or primer

<400>  30
taccgcaagg ttcttcttcg                                                   20


<210>  31
<211>  15
<212>  DNA
<213>  artificial

<220>
<223>  LAMP probe or primer

<400>  31
gagctaccag acgaa                                                        15


<210>  32
<211>  21
<212>  DNA
<213>  artificial

<220>
<223>  LAMP probe or primer

<400>  32
aatgaaagat ctcagtccaa g                                                 21


<210>  33
<211>  15
<212>  DNA
<213>  artificial

<220>
<223>  LAMP probe or primer

<400>  33
agatctcagt ccaag                                                        15


<210>  34
<211>  22
<212>  DNA
<213>  artificial

<220>
<223>  LAMP probe or primer

<400>  34
ggtatttcta ctacctagga ac                                                22


<210>  35
<211>  22
<212>  DNA
<213>  artificial

<220>
<223>  LAMP probe or primer

<400>  35
ccgtctttgt tagcaccata gg                                                22


<210>  36
<211>  44
<212>  DNA
<213>  artificial

<220>
<223>  LAMP probe or primer


<220>
<221>  BIOTIN
<222>  (1)..(1)
<223>  Biotin Label

<400>  36
ccgtctttgt tagcaccata ggggtatttc tactacctag gaac                        44


<210>  37
<211>  17
<212>  DNA
<213>  artificial

<220>
<223>  LAMP probe or primer


<220>
<221>  FAM
<222>  (1)..(1)
<223>  FAM Label

<400>  37
gaagtccagc ttctggc                                                    17


<210>  38
<211>  15
<212>  DNA
<213>  artificial

<220>
<223>  LAMP probe or primer

<400>  38
gcaatgttgt tcctt                                                      15


<210>  39
<211>  15
<212>  DNA
<213>  artificial

<220>
<223>  LAMP probe or primer

<400>  39
ctctgctccc ttctg                                                      15


<210>  40
<211>  16
<212>  DNA
<213>  artificial

<220>
<223>  LAMP probe or primer

<400>  40
actacctagg aactgg                                                     16


<210>  41
<211>  22
<212>  DNA
<213>  artificial

<220>
<223>  LAMP probe or primer

<400>  41
gccagaagct ggacttccct at                                              22


<210>  42
<211>  21
<212>  DNA
<213>  artificial

<220>
<223>  LAMP probe or primer

<400>  42
ccctcagttg caacccatat g                                               21

<210>  43
<211>  19
<212>  DNA
<213>  artificial

<220>
<223>  LAMP probe or primer

<400>  43
tcaaggctcc ctcagttgc                                                    19


<210>  44
<211>  43
<212>  DNA
<213>  artificial

<220>
<223>  LAMP probe or primer


<220>
<221>  BIOTIN
<222>  (1)..(1)
<223>  Biotinlabel

<400>  44
ccctcagttg caacccatat ggccagaagc tggacttccc tat                         43


<210>  45
<211>  41
<212>  DNA
<213>  artificial

<220>
<223>  LAMP probe or primer


<220>
<221>  BIOTIN
<222>  (1)..(1)
<223>  Biotin Label

<400>  45
tcaaggctcc ctcagttgcg ccagaagctg gacttcccta t                           41


<210>  46
<211>  15
<212>  DNA
<213>  artificial

<220>
<223>  LAMP probe or primer


<220>
<221>  FAM
<222>  (1)..(1)
<223>  FAM Label

<400>  46
gccgtctttg ttagc                                                        15


<210>  47
<211>  15
<212>  DNA
<213>  artificial

<220>
<223>  LAMP probe or primer

<400>  47
ggaccccaaa atcag                                                        15


<210>  48
<211>  20
<212>  DNA
<213>  artificial

<220>
<223>  LAMP probe or primer

<400>  48
cgaaatgcac cccgcattac                                                   20


<210>  49
<211>  17
<212>  DNA
<213>  artificial

<220>
<223>  LAMP probe or primer


<220>
<221>  FAM
<222>  (1)..(1)
<223>  FAM Label

<400>  49
gttgaatctg agggtcc                                                      17


<210>  50
<211>  20
<212>  DNA
<213>  artificial

<220>
<223>  LAMP probe or primer

<400>  50
tctccattct ggttactgcc                                                   20


<210>  51
<211>  40
<212>  DNA
<213>  artificial

<220>
<223>  LAMP probe or primer


<220>
<221>  BIOTIN
<222>  (1)..(1)
<223>  Biotin Label

<400>  51
tctccattct ggttactgcc cgaaatgcac cccgcattac                              40


<210>  52
<211>  20
<212>  DNA
<213>  artificial

<220>
<223>  LAMP probe or primer

<400>  52
cgcgatcaaa acaacgtcgg                                                    20


<210>  53
<211>  15
<212>  DNA
<213>  artificial

<220>
<223>  LAMP probe or primer

<400>  53
ccccaaggtt taccc                                                         15


<210>  54
<211>  20
<212>  DNA
<213>  artificial

<220>
<223>  LAMP probe or primer

<400>  54
agcggtgaac caagacgcag                                                    20


<210>  55
<211>  40
<212>  DNA
<213>  artificial

<220>
<223>  LAMP probe or primer

<400>  55
cgcgatcaaa acaacgtcgg agcggtgaac caagacgcag                              40


<210>  56
<211>  16

<212>  DNA
<213>  artificial

<220>
<223>  LAMP probe or primer

<400>  56
cttgccatgt tgagtg                                                    16


<210>  57
<211>  15
<212>  DNA
<213>  artificial

<220>
<223>  LAMP probe or primer

<400>  57
acgttcggat gctcg                                                     15


<210>  58
<211>  20
<212>  DNA
<213>  artificial

<220>
<223>  LAMP probe or primer

<400>  58
actgcacctc atggtcatgt                                                20


<210>  59
<211>  40
<212>  DNA
<213>  artificial

<220>
<223>  LAMP probe or primer


<220>
<221>  DIG
<222>  (1)..(1)
<223>  DIG Label

<400>  59
ctacgaccgt actgaatgcc actgcacctc atggtcatgt                          40


<210>  60
<211>  20
<212>  DNA
<213>  artificial

<220>
<223>  LAMP probe or primer

<400>  60
gtgggcgaaa taccagtggc                                                20

<210>  61
<211>  20
<212>  DNA
<213>  artificial

<220>
<223>  LAMP probe or primer

<400>  61
ccagctcctt tattaccgtt                                                    20


<210>  62
<211>  40
<212>  DNA
<213>  artificial

<220>
<223>  LAMP probe or primer

<400>  62
gtgggcgaaa taccagtggc ccagctcctt tattaccgtt                              40


<210>  63
<211>  15
<212>  DNA
<213>  artificial

<220>
<223>  LAMP probe or primer

<400>  63
gcgccgtaac tatgg                                                         15


<210>  64
<211>  20
<212>  DNA
<213>  artificial

<220>
<223>  LAMP probe or primer

<400>  64
caaagacggc atcatatggg                                                    20


<210>  65
<211>  16
<212>  DNA
<213>  artificial

<220>
<223>  LAMP probe or primer

<400>  65
actgagggag ccttga                                                        16


<210>  66
<211>  20

<212>  DNA
<213>  artificial

<220>
<223>  LAMP probe or primer

<400>  66
ggattgcggg tgccaatgtg                                                    20


<210>  67
<211>  40
<212>  DNA
<213>  artificial

<220>
<223>  LAMP probe or primer

<400>  67
caaagacggc atcatatggg ggattgcggg tgccaatgtg                              40


<210>  68
<211>  16
<212>  DNA
<213>  artificial

<220>
<223>  LAMP probe or primer

<400>  68
agcacgattg cagcat                                                        16


<210>  69
<211>  20
<212>  DNA
<213>  artificial

<220>
<223>  LAMP probe or primer

<400>  69
caactgaggg agccttgaat                                                    20


<210>  70
<211>  15
<212>  DNA
<213>  artificial

<220>
<223>  LAMP probe or primer

<400>  70
tcacattggc acccg                                                         15


<210>  71
<211>  18
<212>  DNA
<213>  artificial

<220>
<223>  LAMP probe or primer

<400>  71
tgcagcattg ttagcagg                                                    18


<210>  72
<211>  38
<212>  DNA
<213>  artificial

<220>
<223>  LAMP probe and primer

<400>  72
caactgaggg agccttgaat tgcagcattg ttagcagg                              38


<210>  73
<211>  16
<212>  DNA
<213>  artificial

<220>
<223>  LAMP probe and primer

<400>  73
gaggaagttg tagcac                                                      16


<210>  74
<211>  18
<212>  DNA
<213>  artificial

<220>
<223>  LAMP probe or primer

<400>  74
agtaccccat cgagcacg                                                    18


<210>  75
<211>  40
<212>  DNA
<213>  artificial

<220>
<223>  LAMP probe or primer


<220>
<221>  DIG
<222>  (1)..(1)
<223>  DIG label

<400>  75
gagccacacg cagctcattg tatcaccaac tgggacgaca                            40


<210>  76
<211>  21

<212>  DNA
<213>  artificial

<220>
<223>  LAMP probe or primer

<400>  76
tgtggtgcca gattttctcc a                                                21


<210>  77
<211>  23
<212>  DNA
<213>  artificial

<220>
<223>  LAMP probe or primer

<400>  77
cgagaagatg acccagatca tgt                                              23


<210>  78
<211>  38
<212>  DNA
<213>  artificial

<220>
<223>  LAMP probe or primer

<400>  78
ctgaacccca aggccaaccg gctggggtgt tgaaggtc                              38


<210>  79
<211>  20
<212>  DNA
<213>  artificial

<220>
<223>  LAMP probe or primer

<400>  79
agcctggata gcaacgtaca                                                  20


<210>  80
<211>  600
<212>  DNA
<213>  SARS CoV-2

<400>  80
ttcgtccgtg ttgcagccga tcatcagcac atctaggttt cgtccgggtg tgaccgaaag      60

gtaagatgga gagccttgtc cctggtttca acgagaaaac acacgtccaa ctcagtttgc     120

ctgttttaca ggttcgcgac gtgctcgtac gtggctttgg agactccgtg gaggaggtct     180

tatcagaggc acgtcaacat cttaaagatg gcacttgtgg cttagtagaa gttgaaaaag     240

gcgttttgcc tcaacttgaa cagccctatg tgttcatcaa acgttcggat gctcgaactg     300

cacctcatgg tcatgttatg gttgagctgg tagcagaact cgaaggcatt cagtacggtc     360

gtagtggtga gacacttggt gtccttgtcc ctcatgtggg cgaaatacca gtggcttacc     420

gcaaggttct tcttcgtaag aacggtaata aaggagctgg tggccatagt tacggcgccg     480

atctaaagtc atttgactta ggcgacgagc ttggcactga tccttatgaa gattttcaag     540

aaaactggaa cactaaacat agcagtggtg ttacccgtga actcatgcgt gagcttaacg     600


<210>  81
<211>  600
<212>  DNA
<213>  SARS CoV-2

<400>  81
aagcaggcac aacgtcggct agtagtcgtg tagatccaaa gcaggcccac actggctttc      60

cattctacct ctcggaacag ggaccaaagt tgctcttttg tgtgcaggtt gagtcaaacg     120

gacaaaatgt ccaagcgctg cacgagcatg caccgaaacc tctgaggcac ctcctccaga     180

atagtctccg tgcagttgta gaatttctac cgtgaacacc gaatcatctt caactttttc     240

cgcaaaacgg agttgaactt gtcgggatac acaagtagtt tgcaagccta cgagcttgac     300

gtggagtacc agtacaatac caactcgacc atcgtcttga gcttccgtaa gtcatgccag     360

catcaccact ctgtgaacca caggaacagg gagtacaccc gctttatggt caccgaatgg     420

cgttccaaga agaagcattc ttgccattat ttcctcgacc accggtatca atgccgcggc     480

tagatttcag taaactgaat ccgctgctcg aaccgtgact aggaatactt ctaaaagttc     540

ttttgacctt gtgatttgta tcgtcaccac aatgggcact tgagtacgca ctcgaattgc     600


<210>  82
<211>  178
<212>  PRT
<213>  SARS CoV-2

<400>  82

Met Glu Ser Leu Val Pro Gly Phe Asn Glu Lys Thr His Val Gln Leu
1               5                   10                  15


Ser Leu Pro Val Leu Gln Val Arg Asp Val Leu Val Arg Gly Phe Gly
            20                  25                  30


Asp Ser Val Glu Glu Val Leu Ser Glu Ala Arg Gln His Leu Lys Asp
        35                  40                  45


Gly Thr Cys Gly Leu Val Glu Val Glu Lys Gly Val Leu Pro Gln Leu
    50                  55                  60


Glu Gln Pro Tyr Val Phe Ile Lys Arg Ser Asp Ala Arg Thr Ala Pro
65                  70                  75                  80

His Gly His Val Met Val Glu Leu Val Ala Glu Leu Glu Gly Ile Gln
                85                  90                  95

Tyr Gly Arg Ser Gly Glu Thr Leu Gly Val Leu Val Pro His Val Gly
            100                 105                 110

Glu Ile Pro Val Ala Tyr Arg Lys Val Leu Leu Arg Lys Asn Gly Asn
        115                 120                 125

Lys Gly Ala Gly Gly His Ser Tyr Gly Ala Asp Leu Lys Ser Phe Asp
    130                 135                 140

Leu Gly Asp Glu Leu Gly Thr Asp Pro Tyr Glu Asp Phe Gln Glu Asn
145                 150                 155                 160

Trp Asn Thr Lys His Ser Ser Gly Val Thr Arg Glu Leu Met Arg Glu
                165                 170                 175

Leu Asn

<210>  83
<211>  178
<212>  PRT
<213>  SARS CoV-2

<400>  83

Met Glu Ser Leu Val Pro Gly Phe Asn Glu Lys Thr His Val Gln Leu
1               5                   10                  15

Ser Leu Pro Val Leu Gln Val Arg Asp Val Leu Val Arg Gly Phe Gly
            20                  25                  30

Asp Ser Val Glu Glu Val Leu Ser Glu Ala Arg Gln His Leu Lys Asp
        35                  40                  45

Gly Thr Cys Gly Leu Val Glu Val Glu Lys Gly Val Leu Pro Gln Leu
    50                  55                  60

Glu Gln Pro Tyr Val Phe Ile Lys Arg Ser Asp Ala Arg Thr Ala Pro
65                  70                  75                  80

His Gly His Val Met Val Glu Leu Val Ala Glu Leu Glu Gly Ile Gln
                85                  90                  95

Tyr Gly Arg Ser Gly Glu Thr Leu Gly Val Leu Val Pro His Val Gly
            100                 105                 110

Glu Ile Pro Val Ala Tyr Arg Lys Val Leu Leu Arg Lys Asn Gly Asn
        115                 120                 125

Lys Gly Ala Gly Gly His Ser Tyr Gly Ala Asp Leu Lys Ser Phe Asp
    130                 135                 140

Leu Gly Asp Glu Leu Gly Thr Asp Pro Tyr Glu Asp Phe Gln Glu Asn
145                 150                 155                 160

Trp Asn Thr Lys His Ser Ser Gly Val Thr Arg Glu Leu Met Arg Glu
                165                 170                 175

Leu Asn

<210>  84
<211>  180
<212>  DNA
<213>  SARS CoV-2

<400>  84
gaccccaaaa tcagcgaaat gcaccccgca ttacgtttgg tggaccctca gattcaactg      60

gcagtaacca gaatggagaa cgcagtgggg cgcgatcaaa acaacgtcgg ccccaaggtt     120

tacccaataa tactgcgtct tggttcaccg ctctcactca acatggcaag gaagacctta     180

<210>  85
<211>  180
<212>  DNA
<213>  SARS CoV-2

<400>  85
ctggggtttt agtcgcttta cgtggggcgt aatgcaaacc acctgggagt ctaagttgac      60

cgtcattggt cttacctctt gcgtcacccc gcgctagttt tgttgcagcc ggggttccaa     120

atgggttatt atgacgcaga accaagtggc gagagtgagt tgtaccgttc cttctggaat     180

<210>  86
<211>  20
<212>  DNA
<213>  artificial

<220>
<223>  T7

<400>  86
taatacgact cactataggg                                                  20

<210>  87
<211>  20
<212>  DNA
<213>  artificial

<220>
<223>  T7

<400>  87
attatgctga gtgatatccc                                                    20


<210>  88
<211>  60
<212>  PRT
<213>  SARS CoV-2

<400>  88

Gly Pro Gln Asn Gln Arg Asn Ala Pro Arg Ile Thr Phe Gly Gly Pro
1               5                   10                  15


Ser Asp Ser Thr Gly Ser Asn Gln Asn Gly Glu Arg Ser Gly Ala Arg
            20                  25                  30


Ser Lys Gln Arg Arg Pro Gln Gly Leu Pro Asn Asn Thr Ala Ser Trp
        35                  40                  45


Phe Thr Ala Leu Thr Gln His Gly Lys Glu Asp Leu
    50                  55                  60


<210>  89
<211>  24
<212>  PRT
<213>  SARS CoV-1

<400>  89

Pro Ser Asp Ser Thr Gly Ser Asn Gln Asn Gly Glu Arg Ser Gly Ala
1               5                   10                  15


Arg Ser Lys Gln Arg Arg Pro Gln
            20


<210>  90
<211>  400
<212>  DNA
<213>  SARS CoV-2

<400>  90
aattccctcg aggacaaggc gttccaatta acaccaatag cagtccagat gaccaaattg        60

gctactaccg aagagctacc agacgaattc gtggtggtga cggtaaaatg aaagatctca       120

gtccaagatg gtatttctac tacctaggaa ctgggccaga agctggactt ccctatggtg       180

ctaacaaaga cggcatcata tgggttgcaa ctgagggagc cttgaataca ccaaaagatc       240

acattggcac ccgcaatcct gctaacaatg ctgcaatcgt gctacaactt cctcaaggaa       300

caacattgcc aaaaggcttc tacgcagaag ggagcagagg cggcagtcaa gcctcttctc       360

gttcctcatc acgtagtcgc aacagttcaa gaaattcaac                    400

<210>  91
<211>  400
<212>  DNA
<213>  SARS CoV-2

<400>  91
ttaagggagc tcctgttccg caaggttaat tgtggttatc gtcaggtcta ctggtttaac      60

cgatgatggc ttctcgatgg tctgcttaag caccaccact gccattttac tttctagagt     120

caggttctac cataaagatg atggatcctt gacccggtct tcgacctgaa gggataccac     180

gattgtttct gccgtagtat acccaacgtt gactccctcg gaacttatgt ggttttctag     240

tgtaaccgtg ggcgttagga cgattgttac gacgttagca cgatgttgaa ggagttcctt     300

gttgtaacgg ttttccgaag atgcgtcttc cctcgtctcc gccgtcagtt cggagaagag     360

caaggagtag tgcatcagcg ttgtcaagtt ctttaagttg                    400

<210>  92
<211>  129
<212>  PRT
<213>  SARS CoV-2

<400>  92

Lys Phe Pro Arg Gly Gln Gly Val Pro Ile Asn Thr Asn Ser Ser Pro
1               5                   10                  15

Asp Asp Gln Ile Gly Tyr Tyr Arg Arg Ala Thr Arg Arg Ile Arg Gly
            20                  25                  30

Gly Asp Gly Lys Met Lys Asp Leu Ser Pro Arg Trp Tyr Phe Tyr Tyr
        35                  40                  45

Leu Gly Thr Gly Pro Glu Ala Gly Leu Pro Tyr Gly Ala Asn Lys Asp
    50                  55                  60

Gly Ile Ile Trp Val Ala Thr Glu Gly Ala Leu Asn Thr Pro Lys Asp
65                  70                  75                  80

His Ile Gly Thr Arg Asn Pro Ala Asn Asn Ala Ala Ile Val Leu Gln
                85                  90                  95

Leu Pro Gln Gly Thr Thr Leu Pro Lys Gly Phe Tyr Ala Glu Gly Ser
            100                 105                 110

Arg Gly Gly Ser Gln Ala Ser Ser Arg Ser Ser Ser Arg Ser Arg Asn
        115                 120                 125

Ser

<210>  93
<211>  22
<212>  PRT
<213>  SARS CoV-2

<400>  93

Glu Pro Ile His Gln Lys Ile Thr Leu Ala Pro Ala Ile Leu Leu Thr
1               5                   10                  15

Met Leu Gln Ser Cys Tyr
            20

<210>  94
<211>  17
<212>  DNA
<213>  artificial

<220>
<223>  LAMP probe or primer

<400>  94
gttgaatctg agggtcc                                                      17

<210>  95
<211>  40
<212>  DNA
<213>  artificial

<220>
<223>  LAMP probe or primer

<400>  95
tctccattct ggttactgcc cgaaatgcac cccgcattac                             40